# EUROPÄISCHE PATENTSCHRIFT

(11) **EP 2 440 119 B1**
(45) Veröffentlichungstag und Bekanntmachung des Hinweises auf die Patenterteilung: **13.05.2015**
(21) Anmeldenummer: 10734055.6
(22) Anmeldetag: 08.06.2010
(51) Int. Cl.: A61B 1/00, A61B 1/05, A61B 1/06, A61B 5/1455, A61B 5/00, G01N 21/64, G02B 21/36, G02B 21/16, H04N 5/225, H04N 5/33, H04N 9/04, A61B 1/04

(54) **BILDGEBUNGSSYSTEM ZUR FLUORESZENZ-OPTISCHEN VISUALISIERUNG EINES OBJEKTS**
IMAGING SYSTEM FOR THE FLUORESCENCE-OPTICAL VISUALIZATION OF AN OBJECT
SYSTÈME D'IMAGERIE DE VISUALISATION D'UN OBJET PAR OPTIQUE DE FLUORESCENCE

(30) Priorität: 10.06.2009 DE 102009024943
(43) Veröffentlichungstag der Anmeldung: 18.04.2012
(73) Patentinhaber: W.O.M. World of Medicine AG, 10587 Berlin (DE)
(72) Erfinder: SCHÖNBORN, Karl-Heinz, 12355 Berlin (DE); BEMBENEK, Andreas, 30175 Hannover (DE); BECKER, Jörn, 12165 Berlin (DE); BOCK, Martin, 13465 Berlin (DE); LUTZ, Andreas, 12623 Berlin (DE)
(74) Vertreter: Schröder, Christoph
(86) Internationale Anmeldenummer: PCT/EP2010/057995
(87) Internationale Veröffentlichungsnummer: WO 2010/142672

(56) Entgegenhaltungen:
- EP-A1- 2 017 591
- US-A- 5 503 904
- US-A- 6 148 227
- US-A1- 2007 015 963
- US-A1- 2008 228 037
- US-B1- 6 826 424
- US-B2- 6 473 176

## Beschreibung

Die Erfindung betrifft ein Bildgebungssystem zur fluoreszenz-optischen Visualisierung eines zweidimensionalen oder dreidimensionalen Objekts nach dem Oberbegriff des Anspruchs 1.

Ein derartiges Bildgebungssystem zur fluoreszenz-optischen Visualisierung eines zweidimensionalen oder dreidimensionalen Objekts, insbesondere des Körpers eines Patienten und seiner Organe und/oder Gewebebereiche, weist eine Beleuchtungseinheit und eine Aufnahmeeinheit auf, die einerseits das Objekt beleuchten, d.h. mit einer optischen Strahlung sichtbaren und/oder infraroten Lichts bestrahlen, und andererseits ein in oder an dem Objekt infolge der Bestrahlung erzeugtes optisches Signal aufnehmen. Die Beleuchtungseinheit ist hierzu ausgebildet, optische Strahlung in einem vorbestimmten Wellenlängenbereich zur Beleuchtung des Objekts und zur Anregung eines in dem Objekt enthaltenen fluoreszierenden Stoffes zu emittieren, während die Aufnahmeeinheit ausgebildet und vorgesehen ist, ein optisches Signal aus dem Bereich des Objekts aufzunehmen und das optische Signal in ein Fluoreszenzsignal mit einem ersten Wellenlängenbereich und ein Signal sichtbaren Lichts mit einem zweiten Wellenlängenbereich aufzuteilen.

Das Fluoreszenzsignal entsteht in einem menschlichen Körper beispielsweise durch Anregung eines geeigneten Kontrastmittels, beispielsweise in Form eines Farbstoffs wie Indocyaningrün (ICG), das einem fluoreszierenden Farbstoff entspricht, der in der Medizin herkömmlich bereits als Indikatorsubstanz (z. B. für die photometrische Leberfunktionsdiagnostik und Fluoreszenzangiographie) bei Herz-, Kreislauf-, Leber- und Augenerkrankungen eingesetzt wird. ICG wird hierzu beispielsweise intravenös oder auch zur Eindiffusion auf der Haut verabreicht und auf natürliche Weise in Abhängigkeit von der Leberleistung mit einer Halbwertszeit von ca. 3-4 Minuten aus dem Körper eliminiert. ICG kann als Natriumsalz in Pulverform vorliegen und kann in unterschiedlichen Lösungsmitteln gelöst werden. Das Absorptions- und Fluoreszenzspektrum von ICG liegt im nahinfraroten Bereich. Das Maximum des Fluoreszenzspektrums liegt unterschiedlich je nach Lösungsmittel: in Blut liegt es bei einer Wellenlänge von etwa 830 nm, in Wasser bei etwa 820 nm (bei einer Anregungswellenlänge von z. B. 765 nm).

Bei einem aus der US2006/0108509 A1 bekannten Bildgebungssystem wird sichtbares Licht zusammen mit einer Anregungsstrahlung im infraroten Bereich auf ein Objekt gestrahlt, und aus dem Bereich des Objekts wird ein optisches Signal aufgenommen. Über Strahlteiler in Form einer Spiegelanordnung wird das optische Signal dann in ein erstes, einem Fluoreszenzsignal entsprechendes Signal im Infrarotbereich und ein zweites Signal im Bereich des sichtbaren Lichts geteilt. Die Signale werden anschließend durch mehrere optoelektronische Wandler in elektronische Datensignale umgewandelt, in einer Verarbeitungseinheit weiterverarbeitet und auf einem Monitor angezeigt.

Bei einem aus der US 6,293,911 B1 bekannten Bildgebungssystem wird in ähnlicher Weise ein Objekt angeregt und ein optisches Signal aus dem Bereich des Objektes aufgenommen. Das optische Signal wird über eine Spiegelanordnung in ein Signal sichtbaren Lichts und ein Fluoreszenzsignal geteilt, wobei das Signal sichtbaren Lichts anschließend unter Verwendung eines dichroitischen Prismas in einen Rot-, einen Grün- und einen Blauanteil (die so genannten RGB-Farben) zerlegt und weiterverarbeitet wird, wie dies beispielsweise auch von Farb-Videokameras bekannt ist.

Sowohl die Anordnung der US2006/0108509 A1 als auch das System der US 6,293,911 B1 verwenden separate Spiegel, um Fluoreszenzsignale von Signalen sichtbaren Lichts zu trennen. Die daraus resultierenden Anordnungen erfordern einen gewissen Bauraum für die Bereitstellung der Spiegel und die Lichtausbreitung zwischen den Spiegeln. Zudem ist eine Erweiterung der Kanäle der Anordnung beispielsweise zur Aufteilung und Verarbeitung weiterer Signale nicht ohne weiteres möglich.

Dokumente US 6 826 424 B1 und US 2008/228037 A1 offenbaren Bildgebungssysteme nach dem Oberbegriff des unabhängigen Anspruchs 1. Der vorliegenden Erfindung liegt die Aufgabe zugrunde, ein Bildgebungssystem und ein Verfahren zur fluoreszenz-optischen Visualisierung eines Objekts, bei denen die Aufnahmeeinheit kompakt aufgebaut und die Kanalzahl zur Verarbeitung unterschiedlicher Signale in einfacher Weise erweitert werden kann.

Diese Aufgabe wird durch ein Bildgebungssystem mit den Merkmalen des Anspruchs 1 gelöst.

Dabei ist vorgesehen, dass die optische Aufnahmeeinheit einen mehrere Teilbereiche aufweisenden optoelektronischen Wandler zur Umwandlung des Fluoreszenzsignals in ein erstes elektronisches Datensignal und des Signals sichtbaren Lichts in ein zweites elektronisches Datensignal aufweist.

Bei einem anspruchsgemäßen Bildgebungssystem wird anstelle mehrerer getrennter optoelektronischer Wandler ein einziger optoelektronischer Wandler verwendet, der mehrere Teilbereiche zur Umwandlung der erzeugten Signale aufweist. Die Aufspaltung des angeregten optischen Signals erfolgt in einem mehrkanaligen dichroitischen Prisma, das das optische Signal in gewünschter Weise auf die genannten Teilbereiche aufteilt. Die aufgeteilten Signale werden dann dem optoelektronischen Wandler zugeführt, dort in elektronische Datensignale gewandelt und beispielsweise an eine Steuer- und Verarbeitungseinheit zur Weiterverarbeitung weitergeleitet.

Durch die Verwendung lediglich eines optoelektronischen Wandlers mit mehreren Teilbereichen vereinfacht sich der Aufbau erheblich. Insbesondere müssen nicht mehrere optoelektronische Wandler parallel vorgesehen und mit Zuleitungen verschaltet werden. Zudem kann sich auch der Signalausleseprozess vereinfachen, da Signale nur durch einen optoelektronischen Wandler erzeugt und entsprechend weiterverarbeitet werden.

Beispielsweise wandelt von den Teilbereichen des optoelektronischen Wandlers ein erster Teilbereich das Fluoreszenzsignal in ein erstes elektronisches Datensignal und ein zweiter Teilbereich das Signal sichtbaren Lichts in ein zweites elektronisches Datensignal um. Es sind damit am optoelektronischen Wandler räumlich getrennte Bereiche vorgesehen, denen die unterschiedlichen Signale zugeführt werden und die die Signale räumlich getrennt wandeln. Eine Trennung des Fluoreszenzsignals von dem Signal sichtbaren Lichts und eine Umwandlung an räumlich voneinander getrennten Teilbereichen ist erforderlich, um das in seiner Intensität sehr viel schwächere Fluoreszenzsignal ungestört von dem Signal sichtbaren Lichts empfangen und mit hinrechender Empfindlichkeit umwandeln zu können.

Durch Verwendung eines mit dem optoelektronischen Wandler verbundenen dichroitischen Prismas zur Aufteilung des aufgenommenen optischen Signals in das Fluoreszenzsignal und das Signal sichtbaren Lichts wird eine Anordnung geschaffen, die auf weitere strahlteilende Spiegel zur Aufteilung des optischen Signals verzichten kann. Das Fluoreszenzsignal und das Signal sichtbaren Lichts werden über ein dichroitisches Prisma voneinander separiert. Auf diese Weise wird ein kompakter Aufbau erhalten, der mit einem einzigen Prisma zur Strahlaufteilung auskommen kann und zudem kurze Ausbreitungswege zwischen Signalaufteilung und Signaldetektion gewährleistet. Das System ist dadurch zum einen vergleichsweise einfach aufzubauen und zudem weniger empfindlich gegenüber Störungen im Betrieb.

Darüber hinaus kann durch Verwendung eines dichroitischen Drei-Kanal- oder Vier-Kanal-Prismas oder eines Prismas mit sogar mehr als vier Kanälen die Kanalzahl des Systems in einfacher Weise skaliert werden, so dass das aufgenommene optische Signal in mehrere unterschiedliche Anteile aufgeteilt werden kann, die anschließend getrennt verarbeitet werden können.

Unter einem dichroitischen Prisma ist in diesem Zusammenhang ein optisches Prisma zu verstehen, das einen Lichtstrahl in mindestens zwei Strahlen unterschiedlicher Spektren, d.h. unterschiedlicher Wellenlängenbereiche, aufteilt. Es wird gewöhnlich aus Glas gefertigt, wobei bestimmte Oberflächen mit dichroitischen Spiegeln versehen sind, die Licht abhängig von dessen Wellenlänge reflektieren oder durchlassen.

Bei einer beispielsweisen Ausführungsform eines dichroitischen Prismas tritt das optische Signal in Form eines Lichtstrahls in ein erstes Teilprisma ein und auf einen ersten dichroitische Filter auf, der einen ersten Anteil des optischen Signals mit einem ersten Wellenlängenbereich reflektiert und das übrige Licht, beispielsweise Licht längerer Wellenlängen, durchlässt. Dieses Licht tritt in ein zweites Teilprisma ein und wird durch einen zweiten dichroitischen Filter geteilt, welcher einen zweiten Anteil des Lichts reflektiert und einen dritten Anteil durchlässt. Die Winkel der einzelnen Teilprismen sind so gewählt, dass der erste und zweite Anteil mittels Totalreflexion in den jeweiligen Teilprismen umgelenkt werden.

Der erste Wellenlängenbereich und der zweite Wellenlängenbereich des Fluoreszenzsignals bzw. des Signals sichtbaren Lichts unterscheiden sich voneinander. Der erste Wellenlängenbereich, der dem Wellenlängenbereich des Fluoreszenzsignals entspricht, kann beispielsweise Wellenlängen größer als 800 nm enthalten und liegt damit im Infrarotbereich. Der zweite Wellenlängenbereich des Signals sichtbaren Lichts kann demgegenüber Wellenlängen kleiner als 700 nm enthalten und liegt damit im Bereich der sichtbaren Wellenlängen.

Der mit dem dichroitischen Prisma verbundene optoelektronische Wandler, beispielsweise ausgebildet als CMOS- oder CCD-Bauelemente, ist vorteilhafterweise unmittelbar auf dem dichroitischen Prisma angeordnet und so mit dem dichroitischen Prisma verbunden, dass die jeweiligen Signale auf den Wandler treffen, dort in elektronische Signale umgewandelt und zur elektronischen Weiterverarbeitung weitergeleitet werden.

Vor dem optoelektronischen Wandler (z. B. in Form eines CCD-Chips) kann zur Detektion des Fluoreszenzsignals zusätzlich ein Langpassfilter angeordnet sein, der nur Wellenlängen größer als die Grenzwellenlänge für das Fluoreszenzsignal (z. B. 800 nm) durchlässt.

Der optoelektronische Wandler kann beispielsweise als Schwarzweiß-Wandler oder als Farb-Wandler ausgebildet sein. Als Schwarzweiß-Wandler kann beispielsweise ein so genannter "S/W-NIR-Enhanced"-Wandler insbesondere für den Empfang von optischen Signalen im (Nah-)Infrarotbereich (NIR: Near Infrared) ausgebildet sein. Als Farb-Wandler kann beispielsweise ein als Bayer-Sensor oder Bayer-Pattern bezeichneter Fotosensor eingesetzt werden, der mit einem Farbfilter ausgestattet ist, der z. B. zu 50% aus Grün, und je 25% aus Rot und Blau besteht (wobei berücksichtigt wird, dass das menschliche Auge auf Grün empfindlicher reagiert als auf andere Farben). Um Farbinformationen zu erhalten, wird dabei in an sich bekannter Weise vor jeder einzelnen Fotozelle des Sensors ein Farbfilter in einer der drei Grundfarben Rot, Grün oder Blau aufgebracht. Jeder Farbpunkt (Pixel) liefert dementsprechend nur Informationen für eine einzige Farbkomponente an dieser Stelle, so dass für ein vollständiges Bild mit denselben Abmessungen die jeweils benachbarten Pixel derselben Farbe zur Farbinterpolation herangezogen werden müssen.

Denkbar ist auch, unterschiedliche Bereiche des optoelektronischen Wandlers unterschiedlich auszubilden, beispielsweise einen ersten Teilbereich zur Umwandlung des Fluoreszenzsignals als Schwarzweiß-Wandler und einen zweiten Teilbereich zur Umwandlung des Signal sichtbaren Lichts als Farbwandler auszubilden.

In einer konkreten Ausgestaltung kann der optoelektronische Wandler eine Anzahl von Pixelelementen aufweisen, die jeweils aus vier Einzelpixeln bestehen, wobei drei Einzelpixel zur Umwandlung des Signals sichtbaren Lichts und ein Einzelpixel zur Umwandlung des Fluoreszenzsignals ausgebildet sind. Der optoelektronische Wandler weist somit eine Anzahl von regelmäßig angeordneten Pixelelementen auf, bestehend jeweils aus vier Einzelpixeln. Von diesen vier Einzelpixeln kann beispielsweise ein erster für rotes Licht empfindlich sein, ein zweiter für grünes Licht und ein dritter für blaues Licht (pro Pixelelement existieren also drei Einzelpixel, die im Bereich des sichtbaren Lichts empfindlich sind), und ein vierter Einzelpixel kann zum Empfang infraroten Lichts ausgebildet sein.

Es ergeben sich somit Pixelelemente, die aus Gruppen von (jeweils vier) Einzelpixeln bestehen und die jeweils für sich sowohl sichtbares Licht als auch infrarotes Licht detektieren können. Dies ermöglicht, den optoelektronischen Wandler als einheitlichen Chip herzustellen, der in unterschiedlichen Bereichen mit unterschiedlichen Signalen bestrahlt wird (beispielsweise in einem ersten Bereich mit dem Fluoreszenzsignal und in einem zweiten Bereich mit dem Signal sichtbaren Lichts) und die Signale dann jeweils in geeigneter Weise umwandelt.

Zusätzlich kann vorgesehen sein, dass die drei Einzelpixel zur Umwandlung des Signals sichtbaren Lichts auch zur Umwandlung des Fluoreszenzsignals ausgebildet sind. Sämtliche Einzelpixel können damit das Fluoreszenzsignal im Bereich des infraroten Lichts empfangen, so dass das (intensitätsschwache) Fluoreszenzsignal von sämtlichen Einzelpixeln detektiert wird. Die Farbanteile des (stärkeren) Signals sichtbaren Lichts (rot, grün und blau) hingegen werden jeweils nur von einem Einzelpixel umgewandelt.

Bei Ausbildung des dichroitischen Prismas als Drei-Kanal-Prisma kann vorgesehen sein, das aufgenommene optische Signal zusätzlich in ein Fluoreszenzanregungssignal mit einem dritten Wellenlängenbereich zu teilen, der von dem ersten Wellenlängenbereich und dem zweiten Wellenlängenbereich unterschiedlich ist und in einem Wellenlängenbereich zwischen 700 nm und 800 nm liegt, also zwischen dem Signal sichtbaren Lichts (mit Wellenlängen kleiner als 700 nm) und dem Fluoreszenzsignal (mit Wellenlängen größer als 800 nm). Dieser Wellenlängenbereich entspricht bevorzugt dem Bereich der von der Beleuchtungseinheit emittierten Strahlung zur Anregung der Fluoreszenz des Objekts bzw. eines in dem Objekt enthaltenen fluoreszierenden Stoffes.

Möglich ist, den dritten Kanal des dichroitischen Prismas mit einem Absorberelement beispielsweise in Form eines Schwarzglases zu verbinden, welches das Fluoreszenzanregungssignal absorbiert. Auf diese Weise wird ein Bildgebungssystem geschaffen, dass lediglich den Anteil des aufgenommenen optischen Signals im sichtbaren Wellenlängenbereich kleiner 700 nm und im infraroten Bereich größer 800 nm zur Informationsgewinnung ausnutzt, den Bereich dazwischen jedoch unterdrückt. Liegt die optische Anregungsstrahlung der Beleuchtungseinheit zur Beleuchtung des Objekts und zur Anregung eines in dem Objekt enthaltenen fluoreszierenden Stoffes gerade in diesem Bereich, weist also Wellenlängen zwischen 700 nm und 800 nm auf, so kann dadurch das Streulicht innerhalb der Aufnahmeeinheit minimiert und der Kontrast der empfangenen Signale verbessert werden.

Alternativ ist aber auch möglich, das dichroitische Prisma mit einem weiteren optoelektronischen Wandler zu verbinden (oder einen weiteren Teilbereich eines einzigen optoelektronischen Wandlers vorzusehen), der das Fluoreszenzanregungssignal in ein drittes elektronisches Datensignal umwandelt. Damit wird die Möglichkeit geschaffen, das Signal im Wellenlängenbereich zwischen 700 nm und 800 nm auszunutzen und daraus zusätzliche Informationen zu gewinnen. Beispielsweise können aus dem aus dem Fluoreszenzanregungssignal gewonnenen dritten elektronischen Datensignal weitere Bildinformationen über das betrachtete Objekt erhalten, es können Informationen über den Signal-zu-Rausch-Abstand gewonnen oder die Aussagekraft von Bildinformationen kann gezielt verbessert werden. Beispielsweise können in Gewebebereichen hoher Absorption nicht-fluoreszierende ("quenched") Absorptionsmechanismen wirken, die nicht in einem aus dem Fluoreszenzsignal erhaltenen Fluoreszenzbild, wohl aber in einem aus dem Fluoreszenzanregungssignal erhaltenen Absorptionsbild detektiert werden können. Zudem sind das Fluoreszenzbild und das Absorptionsbild näherungsweise in ihrer Intensität komplementär. Eine Subtraktion oder Verhältnisbildung der entsprechenden Bilder kann demnach dazu dienen, den Kontrast zu erhöhen. Weiter ist möglich, das Absorptionsbild auf eine signifikante Absorptionslinie zu legen (entsprechend z. B. Haemoglobin) und separat auszuwerten.

Der weitere optoelektronische Wandler ist beispielsweise wie der das Fluoreszenzsignal aufnehmende Wandler als Schwarzweiß-Wandler in Form eines "S/W-NIR-Enhanced"-CCD-Chips ausgebildet.

In einer Ausführungsform kann der weitere optoelektronische Wandler zur Detektion des aus der Anregungsstrahlung resultierenden Fluoreszenzanregungssignals mit einem Strahlabschwächungsfilter zusammenwirken, um das intensitätsstarke Fluoreszenzanregungssignal in geeigneter Weise abzuschwächen, so dass dieses durch den optoelektronischen Wandler verarbeitet werden kann. Der Abschwächungsfilter kann beispielsweise aus einem Filterglas, z. B. einem Grauglasfilter, bestehen.

Anstelle eines weiteren optoelektronischen Wandlers kann auch vorgesehen sein, dass der mehrere Teilbereiche aufweisende optoelektronische Wandler einen dritten Teilbereich aufweist, der das Fluoreszenzanregungssignal in ein drittes elektronisches Datensignal umwandelt. Der optoelektronische Wandler weist somit drei Teilbereiche auf, von denen ein erster das Fluoreszenzsignal, ein zweiter das Signal sichtbaren Lichts und ein dritter das Fluoreszenzanregungssignal detektiert und umwandelt.

Alternativ hierzu ist auch möglich, dass ein Teilbereich des mehrere Teilbereiche aufweisenden optoelektronischen Wandlers sowohl das Signal sichtbaren Lichts als auch das Fluoreszenzanregungssignal aufnimmt und umwandelt. Der optoelektronische Wandler kommt somit mit zwei Teilbereichen aus, von denen ein erster das Fluoreszenzsignal und ein zweiter das Signal sichtbaren Lichts sowie das Fluoreszenzanregungssignal wandelt.

Mit dem bereitgestellten Bildgebungssystem ist es insbesondere möglich, für einen Arzt nicht sichtbare Gefäße, Organe oder Organelle (z. B. zur Darstellung des lymphatischen Systems mit den darin eingebundenen Lympfknoten) intraoperativ zu visualisieren. Das Bildgebungssystem kann dabei einerseits offenchirurgisch zur fluoreszenz-optischen Visualisierung durch Beleuchtung und Aufnahme von außen und andererseits beispielsweise endoskopisch oder kolposkopisch zur fluoreszenz-optischen Visualisierung durch Beleuchtung und Aufnahme im Inneren eines Patienten eingesetzt werden. Dazu kann die Aufnahmeeinheit mit einem Objektiv zur Aufnahme des optischen Signals außerhalb des Objekts und/oder mit einer Aufnahmeeinrichtung zur Aufnahme des optischen Signals aus dem Inneren des Objekts verbunden werden. Die Aufnahmeeinrichtung zur Aufnahme des optischen Signals aus dem Inneren des Objekts kann beispielsweise ein Endoskop, ein Kolposkop, eine in das Objekt invasiv oder nicht-invasiv einführbare oder einbringbare Kamera beispielsweise in Form einer intraoral zu verwendenden Kamera oder in Form einer so genannten, durch einen Patienten in Form einer Pille zu schluckenden "Pill-Cam" sein, wobei auch andere Ausgestaltungen einer in einen Patienten einbringbaren Aufnahmeeinrichtung denkbar sind (die Beleuchtungseinheit kann dabei z. B. in der Pille als chemolumineszent wirkende und beispielsweise durch die Magensäure zu triggernde Lichtquelle realisiert sein). Die Aufnahmeeinheit kann wahlweise mit dem Objektiv zur Aufnahme eines optischen Signals außerhalb des Objekts und/oder mit der beispielsweise endoskopischen Aufnahmeeinrichtung zur Aufnahme des optischen Signals aus dem Inneren des Objekts verbunden werden, wobei die Aufnahmeeinrichtung in einfacher Weise abnehmbar ist. Auf diese Weise wird ein vielseitig einsetzbares Bildgebungssystem geschaffen, das wahlweise offenchirurgisch oder endoskopisch einsetzbar ist und dazu lediglich den Austausch einzelner Zubehörkomponenten erfordert.

Neben der Aufnahmeeinheit weist das Bildgebungssystem eine Beleuchtungseinheit auf, die zur Beleuchtung und Anregung des zu untersuchenden Objekts dient und dazu optische Strahlung in einem vorbestimmten Wellenlängenbereich emittiert. Die Beleuchtungseinheit kann hierzu mit zwei Lichtquellen ausgestattet sein, von denen eine erste Lichtquelle eine Fluoreszenzanregungsstrahlung und eine zweite Lichtquelle Strahlung im Bereich sichtbaren Lichts (Weißlicht) erzeugt. Die von der ersten Lichtquelle erzeugte Fluoreszenzanregungsstrahlung kann beispielsweise in einem Wellenlängenbereich zwischen 700 nm und 800 nm liegen, während die Strahlung sichtbaren Lichts vorzugsweise Wellenlängen kleiner als 700 nm aufweist. Die Lichtquellen können beispielsweise durch Laser oder Leuchtdioden (LEDs) verwirklicht sein, die jeweils angepasst sind, optische Strahlung im gewünschten Wellenlängenbereich mit der erforderlichen Intensität und Charakteristik zu erzeugen.

Die von den beiden Lichtquellen der Beleuchtungseinheit erzeugte Strahlung wird vorteilhafterweise durch ein optisches Koppelelement zur Kopplung der Fluoreszenzanregungsstrahlung und der Strahlung im Bereich sichtbaren Lichts in einen Lichtleiter einkoppelt. Das optische Koppelelement kann zusätzlich dichroitische Filterschichten aufweisen und koppelt die Fluoreszenzanregungsstrahlung und die Strahlung im Bereich des sichtbaren Lichts beispielsweise physikalisch oder durch faseroptische Zusammenführung.

Über den Lichtleiter wird die gekoppelte Strahlung zur Beleuchtung hin zu dem Objekt geleitet, wobei der Lichtleiter mit einem Element zur Anpassung der Abstrahlungscharakteristik der optischen Strahlung zur Beleuchtung des Objektes von außen oder zur Beleuchtung von Bereichen innerhalb des Objektes verbunden sein kann. Beispielsweise kann der Lichtleiter zur Anpassung der Abstrahlungscharakteristik mit einem Diffusor und/oder einem Endoskop verbunden sein, um das Objekt von außen oder von innen zu beleuchten und die optische Strahlung gezielt an den Ort zu führen, an dem das Objekt zur fluoreszenz-optischen Visualisierung beleuchtet und angeregt werden soll. Mit andern Worten wird über den Lichtleiter und gegebenenfalls ein Endoskop oder dergleichen die optische Strahlung gezielt in den Bereich geleitet, aus dem die Aufnahmeeinheit dann die gewünschten optischen Signale empfängt. Beleuchtungseinheit und Aufnahmeeinheit können dabei zur Beleuchtung einerseits und Aufnahme andererseits dasselbe Endoskop verwenden, in dem einerseits ein lichtleitender Kanal für die optische (Beleuchtungs-)Strahlung und ein lichtleitender Kanal für das aufgenommene optische Signal angeordnet sind.

Denkbar und vorteilhaft in diesem Zusammenhang kann sein, das dichroitische Prisma zur Aufteilung des aufgenommenen optischen Signals in das Fluoreszenzsignal und das Signal sichtbaren Lichts unmittelbar in der Spitze eines Endoskops, also an dem in das Objekt einzuführenden Ende des Endoskops, anzuordnen, um das optische Signal unmittelbar dort aufzunehmen, wo es entsteht. Auf diese Weise kann auf einen lichtleitenden Kanal in dem Endoskop zur Leitung des aufgenommenen optischen Signals hin zu dem dichroitischen Prisma verzichtet werden, so dass Signalverluste durch Übertragung vermieden oder zumindest weitestgehend minimiert werden. Das optische Signal wird damit bereits am Orte seiner Entstehung erfasst und an dem dichroitischen Prisma nach der Signalaufteilung unmittelbar in elektronische Datensignale gewandelt, die dann über das Endoskop an eine Steuer- und Verarbeitungseinheit zur weiteren Bildverarbeitung und Analyse übermittelt werden.

Eine Steuer- und Verarbeitungseinheit ist unter anderem vorgesehen, um die Beleuchtungseinheit und/oder die Aufnahmeeinheit und damit die Funktionsweise des Bildgebungssystems zu steuern. Die Steuer- und Verarbeitungseinheit gibt dabei einerseits Parameter für den Betrieb der Beleuchtungseinheit und der Aufnahmeeinheit vor und regelt deren Zusammenwirken, zum andern übernimmt sie die Verarbeitung der über die Aufnahmeeinheit aufgenommenen und in elektronische Datensignale gewandelten Signale.

Zur Bildverarbeitung kann die Steuer- und Verarbeitungseinheit ausgebildet sein, aus den erfassten Signalen ein aus dem Signal sichtbaren Lichts erhaltenes Realbild, ein aus dem Fluoreszenzsignal erhaltenes Fluoreszenzbild und/oder ein aus einem Fluoreszenzanregungssignal erhaltenes Infrarotabsorptionsbild zu erzeugen, die in überlagerter Weise oder nebeneinander ausgegeben und für einen Arzt zur Begutachtung angezeigt werden können. Sollen die erhaltenen Bilder überlagert dargestellt werden, so werden die einander zugeordneten Bilder algorithmisch in analog oder digital vorbehandelter Form miteinander fusioniert. Die Fusionierung erfolgt beispielsweise derart, dass auf dem Realbild mit Falschfarben Bereiche markiert werden, in denen auf dem aus dem Fluoreszenzsignal erhaltenen Fluoreszenzbild ein Signal detektiert wurde. Als eine Möglichkeit für einen Vorbehandlungsalgorithmus des Fluoreszenzbildes wird ein Schwellwert definiert, bei Überschreitung dessen ein Signal auf das Realbild übertragen wird. Im Realbild wird mit einer wählbaren Falschfarbe in Echtzeit der Bereich mit Fluoreszenzintensitäten oberhalb der Schwelle markiert. Dabei kann beispielsweise im Sinne einer Ja-Nein-Entscheidung nur die Tatsache der Fluoreszenzstrahlung oder ein der Signalintensität proportionales Fluoreszenzsignal angezeigt werden. Auch andere Skalierungen, wie logarithmische oder mit einem Maximalwert begrenzte Intensitätsdarstellungen des Fluoreszenzsignals, sind denkbar.

In einer weiteren Ausgestaltung kann das Bildgebungssystem einen Zweiachsenscanner zur Projektion eines erzeugten Fluoreszenzbildes auf das Objekt aufweisen. Der Zweiachsenscanner kann beispielsweise ausgebildet sein, einen Lichtstrahl einer Lichtquelle zur Erzeugung eines Fluoreszenzbildes auf dem Objekt periodisch über das Objekt zu lenken, um auf diese Weise ein Bild auf dem Objekt zu erzeugen, das dem aus dem Fluoreszenzsignal erzeugten Fluoreszenzbild entspricht. Auf diese Weise können beispielsweise auf dem Objekt unmittelbar in Falschfarben solche Bereiche angezeigt werden, in denen ein Fluoreszenzsignal vorhanden ist. Einem Arzt kann so während einer Operation in Echtzeit ein Fluoreszenzbild unmittelbar dort angezeigt werden, wo es herrührt, so dass sogar ein zusätzlicher Monitor zum Anzeigen des Fluoreszenzbildes entfallen könnte.

Zusätzlich oder alternativ kann der Zweiachsenscanner ausgebildet sein, optische Strahlung einer Lichtquelle zur Anregung eines Fluoreszenzsignals auf das Objekt zu lenken und/oder angeregte optische Signale von dem Objekt hin zu einem Detektor zu lenken. Mittels des Zweiachsenscanners kann so eine pixelweise Anregung und/oder Aufnahme erfolgen, wobei als Lichtquelle zur Anregung beispielsweise ein Laser verwendet werden kann.

Eine solche Projektionsvorrichtung ist vorteilhaft mit dem oben beschriebenen Bildgebungssystem kombinierbar, kann grundsätzlich aber auch eigenständig in Zusammenwirken mit beliebigen anderen Bildgebungssystemen betrieben werden.

Ein solches Bildgebungssystem zur fluoreszenz-optischen Visualisierung eines zweidimensionalen oder dreidimensionalen Objekts kann beispielsweise in allgemeiner Weise umfassen:
- eine Beleuchtungseinheit, die ausgebildet und vorgesehen ist, optische Strahlung in einem vorbestimmten Wellenlängenbereich zur Beleuchtung des Objekts und zur Anregung eines in dem Objekt enthaltenen fluoreszierenden Stoffes zu emittieren,
- eine Aufnahmeeinheit, die ausgebildet und vorgesehen ist, ein Fluoreszenzsignal aus dem Bereich des Objekts aufzunehmen,
wobei einen Zweiachsenscanner zur Projektion eines aus dem Fluoreszenzsignal erzeugten Fluoreszenzbildes auf das Objekt vorgesehen ist.

Das Bildgebungssystem kann einen Lichtstrahl einer Lichtquelle zur Erzeugung eines Fluoreszenzbildes auf dem Objekt periodisch über das Objekt lenken, wobei die Anregung des Fluoreszenzsignals und die Abbildung des aus dem Fluoreszenzsignal erhaltenen Fluoreszenzbildes vorzugsweise abwechselnd, also nach Art eines Zeitmultiplexes, erfolgen können.

Es kann hierbei auch vorgesehen sein, dass der Zweiachsenscanner zusätzlich zur Anregung und Aufnahme des Fluoreszenzsignals ausgebildet ist und damit auch die Beleuchtungseinheit sowie die Aufnahmeeinheit verwirklicht. Mittels des Zweiachsenscanners wird abwechselnd in einem ersten Zeitfenster ein Signal angeregt und empfangen und in einem zweiten Zeitfenster ein daraus generiertes Fluoreszenzbild auf dem Objekt - also eben dort, wo es generiert worden ist - abgebildet.

Alternativ können die Schritte (a) Fluoreszenzanregung, (b) Fluoreszenzstrahlungsmessung und (c) Projektion eines sichtbaren, mit dem gemessenen Fluoreszenzsignal monoton wachsenden Anzeige-Projektionsstrahls (Fluoreszenzbildprojektion) in unmittelbarer zeitlicher Folge, quasi gleichzeitig, ausgeführt werden. In vorteilhafter Weise wird so mit minimalem zeitlichem und technischem Aufwand eine Aufnahme und Abbildung erreicht.

Eine solche Anordnung eignet sich insbesondere für offenchirurgische Anwendungen.

Bei einem Verfahren zur fluoreszenz-optischen Visualisierung eines zweidimensionalen oder dreidimensionalen Objekts
- wird eine optische Strahlung in einem vorbestimmten Wellenlängenbereich zur Beleuchtung des Objekts und zur Anregung eines in dem Objekt enthaltenen fluoreszierenden Stoffes emittiert und
- wird ein optisches Signal aus dem Bereich des Objekts aufgenommen und das optische Signal in ein Fluoreszenzsignal mit einem ersten Wellenlängenbereich und ein Signal sichtbaren Lichts mit einem zweiten Wellenlängenbereich aufgeteilt.

Bei dem Verfahren ist vorgesehen, dass ein dichroitisches Prisma das aufgenommene optische Signal in das Fluoreszenzsignal und das Signal sichtbaren Lichts teilt und ein mit dem dichroitischen Prisma verbundener, mehrere Teilbereiche aufweisender optoelektronischer Wandler mit einem ersten Teilbereich das Fluoreszenzsignal in ein erstes elektronisches Datensignal und mit einem zweiten Teilbereich das Signal sichtbaren Lichts in ein zweites elektronisches Datensignal umwandelt.

Für das Verfahren ergeben sich die gleichen Vorteile wie oben für das Bildgebungssystem beschrieben.

Das beschriebene Bildgebungssystem und das Verfahren können in einem weiten Gebiet insbesondere für medizinische Zwecke eingesetzt werden. Beispielsweise zählen hierzu das Auffinden von bestimmten signifikanten Organen, Organellen oder Organteilen oder von pathologisch veränderten Gewebebereichen (Lymphknoten, Lymphbahnen, Adern, Gallengänge, Hohlräumen, Entzündungsherden oder dergleichen) im menschlichen Körper mit Hilfe von in den Körper eingebrachten Farbstoffen und deren bildlicher Nachweis mittels fluoreszenz-optischer Methoden und die Beobachtung von Stofftransportphänomenen in körpereigenen Fluss-Systemen (Adern, Lymphbahnen, Hautperfusion und andere) und/oder die qualitative und quantitative Bestimmung von Transportgeschwindigkeiten und -routen sowie Anhäufungsarealen und -volumina dieser Flusssysteme.

Konkret sind folgende medizinische Einsatzzwecke denkbar, wobei die Aufzählung in keiner Weise abschließend zu verstehen sein soll:
- Visualisierung des lymphatischen Systems mit dem Ziel einer Lymphographie (minimal invasiv),
- Sentinel-Lymphknoten-Biopsie (z. B. bei Brust, malignen Melanomen, Lunge, Magen, Darm, Prostata, Zervix, Endometrium),
- Visualisierung des Blutgefäßsystems (Perfusionsmessung) mit dem Ziel der Anastomosekontrolle,
- Schweregradbestimmung bei Verbrennungen (minimal invasiv),
- Bestimmung eines notwendigen Amputationslevels (minimal invasiv),
- Qualitätskontrolle bei Rekonstruktionsoperationen, plastischen Korrekturen oder Gewebetransplantationen,
- Bewertung des "Diabetischen Fuß"- Syndroms (minimal invasiv),
- Dekubituskontrolle (minimal invasiv),
- Darstellung so genannter "bleeding points" (Blutungen) im Rahmen von endoskopischen Operationen,
- Differenzierung zwischen Gewebearten (z.B. Adenom vs. Tumor),
- Erlangung von Informationen über die Invasionstiefe erkannter Tumoren (submukosaler Effekt),
- Visualisierung von Gallengängen im Rahmen der Leber- oder Gallenchirurgie mit dem Ziel der Schonung wichtiger Strukturen,
- Visualisierung von Entartungen zur Krebsvorsorge (so genanntes "screening") und der Früherkennung von Tumoren (z. B. in Zusammenhang mit selektiven Tumormarkern) (minimal invasiv).

Unter "minimal invasiv" ist hier zu verstehen, dass die so gekennzeichneten Einsatzzwecke mindestens den Einsatz eines in einen Körper eingebrachten fluoreszierenden Kontrastmittels erforderlich machen, um ein Fluoreszenzsignal aus dem Körper zu erhalten.

Grundsätzlich wird dabei so vorgegangen, dass ein in den Köper eines Patienten, ob systemisch (durch Einspritzen) oder topisch (durch Aufbringen auf die Körperoberfläche und Eindiffusion) eingebrachter Farbstoff (oder der nach dem Einbringen im Körper sich bildenden Agglomerationen desselben mit körpereigenen Stoffen) durch von der Beleuchtungseinheit emittierte Strahlung zur Fluoreszenz angeregt wird, das resultierende Fluoreszenzsignal über die Aufnahmeeinheit detektiert wird und ein daraus erzeugtes Fluoreszenzbild in einer Weise angezeigt wird, die es dem Mediziner erlaubt, diagnostische oder therapeutische Entscheidungen zu treffen.

Der der Erfindung zugrunde liegende Gedanke soll nachfolgend anhand der in den Figuren dargestellten Ausführungsbeispiele näher erläutert werden. Es zeigen:
- Fig. 1: eine schematische Ansicht einer ersten Ausführungsform eines Bildgebungssystems mit einer Beleuchtungseinheit und einer Aufnahmeeinheit, bei der über ein dichroitisches Prisma ein aufgenommenes optisches Signal in ein Fluoreszenzsignal und ein Signal sichtbaren Lichts geteilt wird (2-Kanal-Variante);
- Fig. 2: eine Detaildarstellung der Aufnahmeeinheit des Bildgebungssystems gemäß Fig. 1;
- Fig. 3: eine Detaildarstellung einer Ausführungsform eines dichroitischen Prismas;
- Fig. 4: eine schematische Ansicht einer zweiten Ausführungsform eines Bildgebungssystems mit einer Beleuchtungseinheit und einer Aufnahmeeinheit, bei der über ein dichroitisches Prisma ein aufgenommenes optisches Signal in ein Fluoreszenzsignal, ein Signal sichtbaren Lichts und ein Fluoreszenzanregungssignal geteilt wird (3-Kanal-Variante);
- Fig. 5: eine Detaildarstellung der Aufnahmeeinheit des Bildgebungssystems gemäß Fig. 4;
- Fig. 6: eine Darstellung eines 4-Kanal-Prismas zur Aufteilung eines optischen Signals in vier Signalanteile;
- Fig. 7: eine schematische Ansicht einer Anordnung zur Aufteilung eines optischen Signals in zwei Signalanteile und der Detektion mittels eines einzigen CCD-Chips;
- Fig. 8A, 8B: Ansichten einer Anordnung zur Detektion eines geteilten optischen Signals mittels eines einzigen CCD-Chips;
- Fig. 9: eine schematische Darstellung einer Anordnung zur Projektion eines Fluoreszenzbildes auf ein Objekt;
- Fig. 10: eine schematische Darstellung einer Anordnung zur Aufnahme und zur Projektion eines Fluoreszenzbildes auf ein Objekt;
- Fig. 11: eine schematische Darstellung einer weiteren Anordnung zur Aufnahme und zur Projektion eines Fluoreszenzbildes auf ein Objekt;
- Fig. 12: eine schematische Ansicht eines CCD-Chips zur Aufnahme sichtbaren Lichts und Infrarotlichts und
- Fig. 13: eine schematische Ansicht einer Filteranordnung des CCD-Chips gemäß Fig. 12.

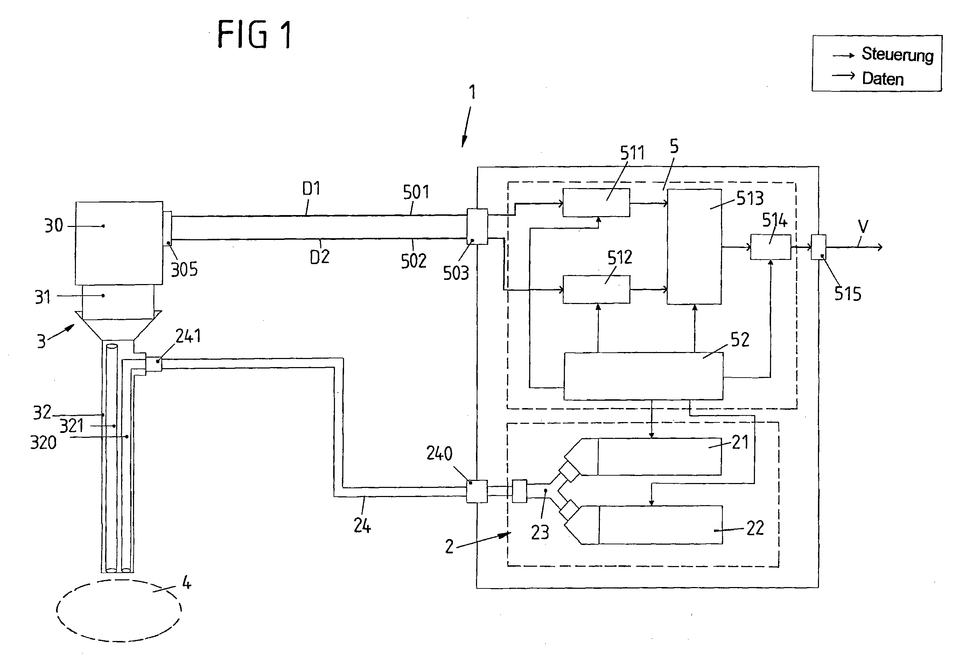

Fig. 1 zeigt zunächst in einer Übersichtsdarstellung eine Ausführungsform eines Bildgebungssystems 1, das eine Beleuchtungseinheit 2 und eine Aufnahmeeinheit 3 umfasst. Das Bildgebungssystem 2 dient zur fluoreszenz-optischen Visualisierung eines Objekts 4, beispielsweise von Gefäßen, Organen oder Organellen eines Patienten, und erzeugt hierzu eine optische Strahlung, mit der das Objekt 4 bestrahlt und zur Emission von fluoreszenz-optischen Signalen angeregt wird.

Grundlegend wird zur fluoreszenz-optischen Visualisierung folgendermaßen vorgegangen. Zunächst wird ein geeigneter fluoreszierender Farbstoff (beispielsweise Indocyaningrün (ICG)), in das Objekt 4, beispielsweise den Körper eines Patienten, eingebracht. Dieses kann systemisch durch Einspritzen oder topisch durch Aufbringen auf die Objektoberfläche und Eindiffusion erfolgen. Anschließend wird der eingebrachte Farbstoff mittels der Beleuchtungseinheit 2 zur Fluoreszenz angeregt, und die resultierenden Signale werden mittels der Aufnahmeeinheit 3 detektiert. Die detektierten Signale werden weiterverarbeitet und in geeigneter Weise beispielsweise als Videosignal in Echtzeit zur Begutachtung für einen Mediziner angezeigt.

Zu betonen ist an dieser Stele, dass die Funktionsweise des Bildgebungssystems von der Art und Weise des Einbringens des fluoreszierenden Stoffes in das Objekt 4 unabhängig ist. Wesentlich ist lediglich, dass in dem Objekt 4 ein Stoff vorhanden ist, der zur Fluoreszenz angeregt werden kann. Grundsätzlich kann dieser Stoff auch auf natürliche Weise in dem Objekt 4 vorhanden sein, ohne dass ein Einbringen von außen erforderlich ist.

Die Beleuchtungseinheit 2 weist zwei Lichtquellen 21, 22 auf, die mittels Lasern oder Leuchtdioden (LEDs) einerseits eine Fluoreszenzanregungsstrahlung in einem Wellenlängenbereich zwischen 700 nm und 800 nm und andererseits eine Strahlung im Bereich sichtbaren Lichts mit Wellenlängen kleiner als 700 nm erzeugen. Die von den Lichtquellen 21, 22 erzeugte Strahlung wird mittels eines optischen Koppelelementes 23 beispielsweise in Form eines physikalischen oder faseroptischen Kopplers gekoppelt und über einen Anschluss 240 in einen Lichtleiter 24, beispielsweise eine flexible optische Faser, eingespeist.

Über den Lichtleiter 24 wird die erzeugte optische Strahlung hin zu einem mit der Aufnahmeeinheit 3 verbundenen Endoskop 32 geleitet und ist über einen Anschluss 241 mit dem Endoskop 32 verbunden. Das Endoskop 32 verfügt über einen lichtleitenden optischen Kanal 320, in dem die optische Strahlung der Beleuchtungseinheit 2 hin zu dem Objekt 4 geführt wird.

Das Endoskop 32 ist zum Einführen in das Objekt 4 ausgebildet. Beispielsweise kann das Endoskop 32 invasiv in den Körper eines Patienten eingeführt werden, um auf diese Weise Gefäße oder Organe des Patienten unmittelbar im Inneren des Patienten anzuregen. Das Endoskop 32 kann in an sich bekannter Weise zumindest abschnittsweise flexibel ausgebildet sein und ermöglicht damit einen einfachen Zugang in das Innere des Objekts 4.

Das Endoskop 32 weist einen zweiten Kanal 321 auf, über den in oder an dem Objekt 4 aus der optischen Anregung resultierende optische Signale aufgenommen und an die Aufnahmeeinheit 3 geleitet werden. Das Endoskop 32 ist hierzu über ein einen Schnellverschluss aufweisendes Objektiv 31 an einen Kamerakopf 30 der Aufnahmeeinheit 3 angeschlossen, in dem die empfangenen optischen Signale zerlegt und gewandelt werden.

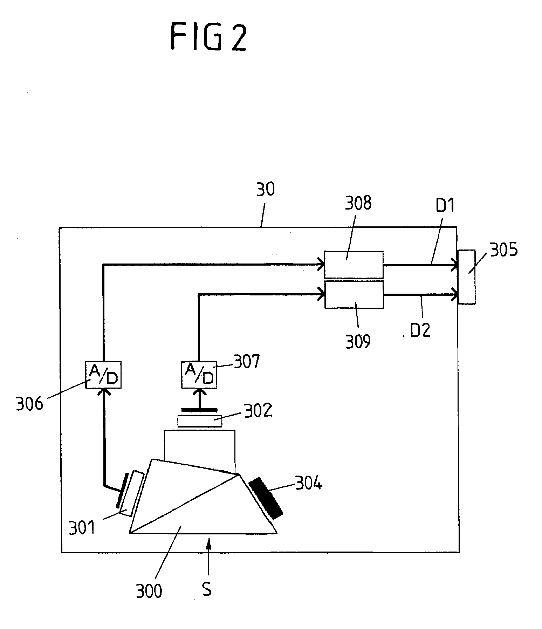

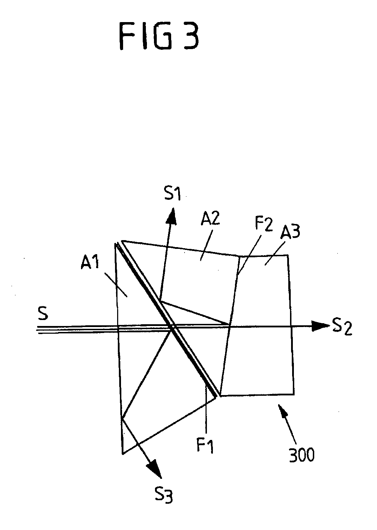

Eine Detailansicht des Kamerakopfs 30 ist in Fig. 2 dargestellt. In dem Kamerakopf 30 ist ein dichroitisches Prisma 300 angeordnet, das mit zwei optoelektronischen Wandlern 301, 302 in Form von CCD-Chips oder CMOS-Bauelementen und zudem mit einem Absorberelement 304 beispielsweise in Form eines Schwarzglases verbunden ist. Das dichroitische Prisma 300 dient der Strahlaufteilung eines empfangenen und auf das dichroitische Prisma 300 treffenden optischen Signals S und zerlegt dieses, wie nachfolgend anhand von Fig. 3 noch erläutert werden soll, in Signalanteile S1, S2, S3, die einem Fluoreszenzsignal mit Wellenlängen größer als 800 nm, einem Signal sichtbaren Lichts mit Wellenlängen kleiner 700 nm einem Fluoreszenzanregungssignal mit Wellenlängen zwischen 700 nm und 800 nm entsprechen. Von diesen Signalanteilen werden lediglich das Fluoreszenzsignal S1 und das Signal sichtbaren Lichts S2 bei dem Aufbau gemäß Fig. 2 über je einen optoelektronischen Wandler 301, 302 detektiert und über Analog/Digital-Wandler 306, 307 und elektronische Treiber 308, 309 in elektronische Datensignale D1, D2 umgewandelt. Das Fluoreszenzanregungssignal S3 hingegen wird über das Absorberelement 304 absorbiert und keiner Bildverarbeitung zugeführt.

Wie aus Fig. 1 ersichtlich, ist der Kamerakopf 30 über einen Anschluss 305 an Datenkabel 501, 502 angeschlossen, über die die elektronischen Datensignale D1, D2 an eine Steuer- und Verarbeitungseinheit 5 geleitet werden. Die Datenkabel 501, 502 sind hierzu über einen Anschluss 503 mit der Steuer- und Verarbeitungseinheit 5 verbunden, die in einem selben Gehäuse wie die Beleuchtungseinheit 2 angeordnet ist.

Die Steuer- und Verarbeitungseinheit 5 dient einerseits der Steuerung der Beleuchtungseinheit 2 und der Aufnahmeeinheit 3 und andererseits der Bildverarbeitung der empfangenen Datensignale D1, D2. Zu diesem Zweck weist die Steuer- und Verarbeitungseinheit 5 Vorverarbeitungseinheiten 511, 512 auf, die zur Vorverarbeitung des dem Fluoreszenzsignal zugeordneten elektronischen Datensignals D1 und des dem Signal sichtbaren Lichts zugeordneten elektronischen Datensignals D2 dienen. Die vorverarbeiteten Datensignale werden an eine Bildverarbeitungseinheit 513 weitergeleitet, die algorithmisch aus den Datensignalen D1, D2 ein dem Fluoreszenzsignal entsprechendes Fluoreszenzbild und ein dem Signal sichtbaren Lichts zugeordnetes Realbild erzeugt.

Die Bildverarbeitungseinheit 513 kann das Fluoreszenzbild und das Realbild beispielsweise miteinander fusionieren, das heißt, so überlagern, dass in einem fusionierten Bild auf dem Realbild solche Bereiche in Falschfarben angezeigt werden, in denen ein Fluoreszenzsignal vorhanden ist. Das so erzeugte Bild wird an eine Endverarbeitungseinheit 514 weitergeleitet, die das so genannte "Framing" zur Echtzeitdarstellung der erzeugten Bilder vornimmt und über einen Anschluss 515 ein Videoausgabesignal V zum Anzeigen auf einem Monitor und zur Begutachtung für einen Mediziner ausgibt.

Die Steuer- und Verarbeitungseinheit 5 verfügt über eine Steuereinheit 52, die die einzelnen Einheiten 511, 512, 513, 514 steuert und gleichzeitig auch die Beleuchtungseinheit 2 insbesondere unter Auswertung der empfangenen Datensignale D1, D2 regelt.

Eine Detailansicht einer Ausführungsform eines dichroitischen Prismas 300 ist in Fig. 3 dargestellt, wobei grundlegend auch andere Ausführungsformen Verwendung finden können. Das dichroitische Prisma 300 weist drei Teilprismen A1, A2, A3 auf, die über dichroitische Filter F1, F2 optisch miteinander gekoppelt sind. Das dichroitische Prisma 300 dient der Aufteilung eines einfallenden optischen Signals S in drei Teilsignale entsprechend einem Fluoreszenzsignal S1, einem Signal sichtbaren Lichts S2 und einem Fluoreszenzanregungssignal S3. Das einfallende optische Signal S trifft dabei zunächst auf die mit dem dichroitischen Filter F1 versehene Grenzfläche des Teilprismas A1 und wird an dieser Grenzfläche teilweise reflektiert und teilweise durchgelassen. Der dichroitische Filter F1 ist so ausgebildet, dass lediglich der Anteil des optischen Signals S im Wellenlängenbereich zwischen 700 nm und 800 nm reflektiert wird, die übrigen Anteile jedoch durchgelassen werden. Die übrigen Anteile treten so in das Teilprisma A2 ein und werden an der mit dem dichroitischen Filter F2 versehenen Grenzfläche des Teilprismas A2 teilweise reflektiert und teilweise durchgelassen. Der dichroitische Filter F2 ist so beschaffen, dass er Signale mit Wellenlängen kleiner 700 nm durchlässt, Signale mit größeren Wellenlängen jedoch reflektiert. Auf diese Weise wird das Fluoreszenzsignal S1 von dem Signal sichtbaren Lichts S2 separiert, wobei das Signal sichtbaren Lichts S2 in das Teilprisma A3 eintritt und dieses passiert, während das Fluoreszenzsignal S1 unter Totalreflektion innerhalb des Teilprismas A2 erneut reflektiert und emittiert wird.

Wie aus Fig. 2 ersichtlich, wird das Fluoreszenzsignal S1 und das Signal sichtbaren Lichts S2 von je einem optoelektronischen Wandler 301, 302 detektiert und umgewandelt, während das Fluoreszenzanregungssignal S3 bei dem Bildgebungssystem gemäß Fig. 1 absorbiert und nicht weiterverarbeitet wird.

Die Verwendung eines dichroitischen Prismas 300 ermöglicht einen kompakten Aufbau des Bildgebungssystems 1 mit seiner Aufnahmeeinheit 3. Insbesondere werden keine separaten Spiegel zur Strahlaufteilung benötigt. Die Strahlaufteilung erfolgt vollständig über ein einheitliches, kompaktes dichroitisches Prisma.

Das Bildgebungssystem 1 kann wahlweise endoskopisch zur Anregung fluoreszenzoptischer Signale innerhalb eines Objekts 4, wie in Fig. 1 dargestellt, oder auch offenchirurgisch zur äußeren Beleuchtung und Aufnahme von fluoreszenz-optischen Signalen außerhalb eines Objekts 4 ohne Verwendung eines Endoskops 32 eingesetzt werden. Das Endoskop 32 kann hierzu über einen Schnellverschluss von dem Objektiv 31 gelöst werden. Zur offenen Anwendung kann dann der Lichtleiter 24 beispielsweise mit einem Diffusor zur Anpassung der Abstrahlungscharakteristik und zur äußeren Beleuchtung des Objekts 4 verbunden werden.

Eine Verwendung eines Diffusors zur Anpassung der Abstrahlungscharakteristik ist grundlegend auch in Zusammenhang mit einem Endoskop 32 denkbar.

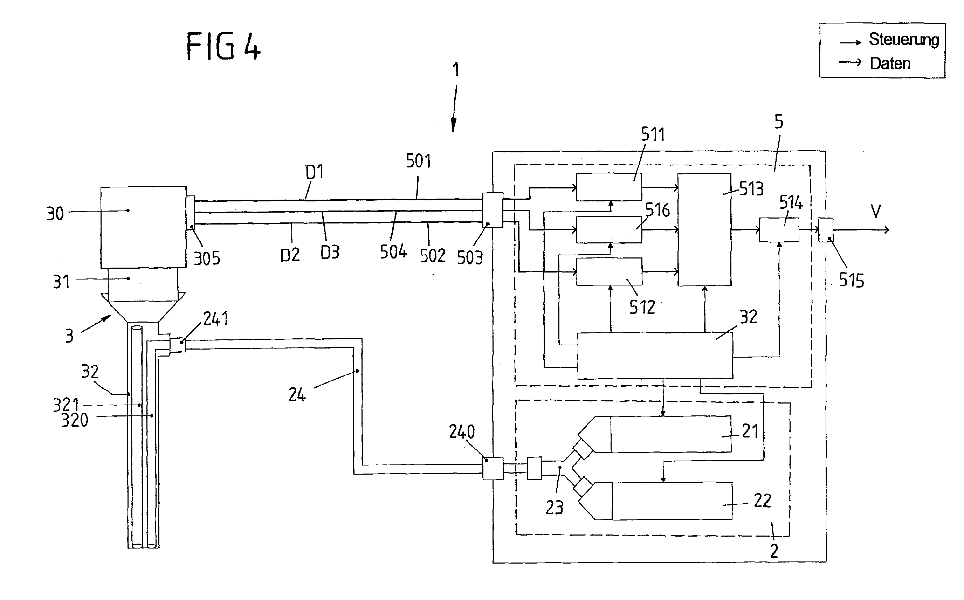

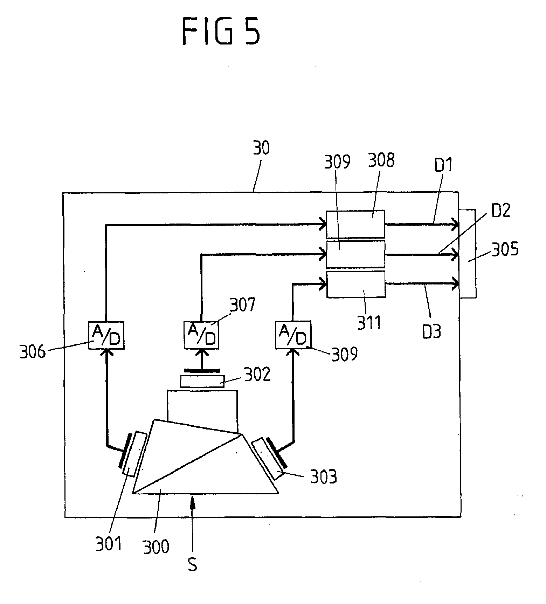

Eine weitere Ausführungsform eines Bildgebungssystems 1 ist in Fig. 4 und 5 dargestellt. Der Aufbau entspricht grundlegend dem Aufbau des Bildgebungssystems 1 gemäß Fig. 1 bis 3, wobei gleiche Bauteile soweit zweckdienlich auch mit gleichen Bezugszeichen bezeichnet sind.

Im Unterschied zu dem Bildgebungssystem 1 gemäß Fig. 1 bis 3 ist bei dem Bildgebungssystem 1 gemäß Fig. 4 und 5 das dichroitische Prisma 300 der Aufnahmeeinheit 3 nicht mit einem Absorberelement 304 (vergleiche Fig. 2), sondern mit einem dritten optoelektronischen Wandler 303 in Form eines CCD-Chips oder CMOS-Bauelementes verbunden (siehe Fig. 5). Das in dem dichroitischen Prisma 300 separierte Fluoreszenzanregungssignal S3 (siehe Fig. 3) wird damit in dem Aufbau gemäß Fig. 4 und 5 nicht unterdrückt, sondern separat detektiert und über einen Anlog/Digital-Wandler 309 und einen Treiber 311 in ein drittes elektronisches Datensignal D3 umgewandelt, das über einen Anschluss 305, eine Datenleitung 504 und den Anschluss 503 der Steuer- und Verarbeitungseinheit 5 zugeführt wird. In einem separaten Kanal wird das dritte elektronische Datensignal D3 über eine zusätzliche Vorverarbeitungseinheit 516 vorverarbeitet und der Bildverarbeitungseinheit 513 zugeführt, die das zusätzliche Signal in geeigneter Weise verarbeitet, daraus Informationen ableitet und beispielsweise ein aus dem Fluoreszenzanregungssignal S3 abgeleitetes Infrarotabsorptionsbild generiert und dem Realbild und/oder dem Fluoreszenzbild überlagert.

Die Anordnung gemäß Fig. 4 und 5 schafft damit ein Bildgebungssystem 1 mit drei Kanälen und ermöglicht die Auswertung zusätzlicher, aus dem Fluoreszenzanregungssignal abzuleitender Informationen. Beispielsweise können aus einem Infrarotabsorptionsbild Gewebebereiche erfasst werden, in denen es aufgrund nicht fluoreszierender Absorptionsmechanismen zu einer hohen Absorption kommt und die nicht im Fluoreszenzbild, wohl aber im Infrarotabsorptionsbild detektiert werden können. Zudem kann durch Subtraktion oder Verhältnisbildung des Infrarotabsorptionsbildes mit dem Fluoreszenzbild ein Rückschluss auf den Signal-zu-Rausch-Abstand gezogen werden. Auch ist möglich, das Infrarotabsorptionsbild auf eine signifikante Absorptionslinie zu legen, die beispielsweise Hämoglobin entspricht, und dieses Infrarotabsorptionsbild separat auszuwerten.

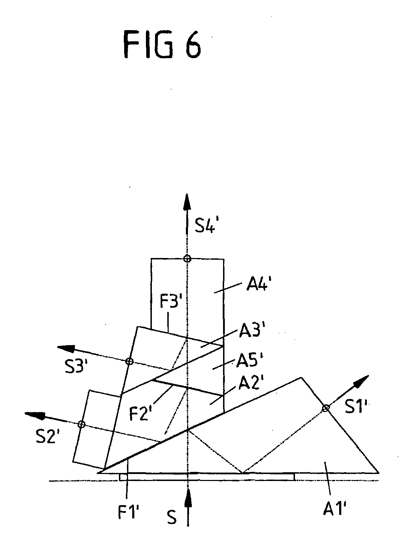

Grundlegend denkbar ist auch, andere dichroitische Prismen zu verwenden und unter Verwendung dieser Prismen eine Aufteilung des optischen Signals S in weitere Signalanteile vorzunehmen. Fig. 6 zeigt beispielsweise ein Vier-Kanal-Prisma, das unter Verwendung von Teilprismen A1', A2', A3', A4', A5', dichroitischen Filtern F1', F2', F3' das optische Signal S in Teilsignale S1', S2', S3', S4' aufteilt, die separat voneinander ausgewertet und bearbeitet werden können. Die Teilsignale S1', S2', S3', S4' können dabei unterschiedlichen Wellenlängenbereichen zugeordnet sein. Denkbar ist beispielsweise, mittels des Vier-Kanal-Prismas das optische Signale S in drei Farbsignale (Grundfarben Rot, Grün, Blau) und ein Fluoreszenzbild aufzuteilen.

Denkbar ist auch, das optische Signal S so aufzuteilen, dass man ein Realbild, ein Fluoreszenzbild, ein Infrarotabsorptionsbild und ein Schmalbandbild (so genanntes "Narrow Band Imaging") erhält.

Mittels dem so genannten "Narrow Band Imaging" kann beispielsweise die Sichtbarkeit von Kapillaren, Venen und anderen feinen Gewebestrukturen verbessert werden. Hierzu werden diskrete Wellenlängen eingesetzt, um Absorption und Reflektion zu vermindern, wobei zum einen Blau (415 nm), um die oberflächlichen Kapillaren darzustellen, und andererseits Grün (540 nm), um tiefer liegende Gefäße darzustellen, verwendet wird. Kombiniert ergibt sich daraus ein Bild der oberen Gewebeschichten mit sehr hohem Kontrast.

In einer ersten Anwendung eines Vier-Kanal-Prismas werden beispielsweise die drei Grundfarben Rot, Grün, Blau unter Verwendung jeweils eines Schwarzweiß-Wandlers detektiert, während im vierten Kanal das Fluoreszenzsignal mit Wellenlängen größer 800 nm detektiert wird.

In einer zweiten Anwendung eines Vier-Kanal-Prismas wird ein Farb-Wandler für das Realbild eingesetzt, das Fluoreszenzbild liefert ein Schwarzweiß-"NIR-Enhanced"-Wandler, das Infrarotabsorptionsbild wird ebenfalls über einen Schwarzweiß-"NIR-Enhanced"-Wandler erhalten und ein vierter Wandler arbeitet in einem "Narrow Band Imaging"-Modus.

In einer dritten Anwendung eines Vier-Kanal-Prismas wird ein Farbsensor für das Realbild eingesetzt, ein Schwarzweiß-"NIR-Enhanced"-Wandler für das Fluoreszenzbild, während ein dritter Wandler das blaue Band (415 nm) und ein vierter Sensor das grüne Band (540 nm) für das so genannte "Narrow Band Imaging" liefert.

Grundlegend ist auch bei Verwendung eines Vier-Kanal-Prismas möglich, einen oder mehrere der Signalanteile unter Verwendung eines oder mehrerer Absorberelemente zu absorbieren, um dadurch Kontrast und Signal-zu-Rausch-Abstand zu verbessern.

In einer weiterführenden Ausgestaltung eines Bildgebungssystems ist auch denkbar, Anregung und Aufnahme des optischen Signals S in einem Zeitmultiplex vorzunehmen, um auf diese Weise zusätzlich zur oder anstelle der Strahlaufteilung mittels des dichroitischen Prismas eine zeitliche Separation des Fluoreszenzsignals S1 und des Signals sichtbaren Lichts S2 zu erreichen. Dazu können zeitlich abwechselnde Bildaufnahmen des Realbildes und des Fluoreszenzbildes über denselben Sensor erfolgen. Realbild und Fluoreszenzbild werden dann getrennt, indem bildsynchron die beiden Lichtquellen 21, 22 für die Strahlung im Bereich des sichtbaren Lichts und für die Fluoreszenzanregungsstrahlung synchron mit der entsprechenden Bildaufnahme für den Realbild- und den Fluoreszenzbildkanal getaktet werden.

Zwei Ansätze sind dabei möglich:
1. Wechselseitige An- und Abschaltung der Lichtquellen 21, 22 für die Fluoreszenzanregungsstrahlung und die Strahlung sichtbaren Lichts;
2. Taktung ausschließlich der Fluoreszenzanregungsstrahlung und Berechnung des Fluoreszenzbildes als Differenz zwischen dem Bild mit und ohne Fluoreszenzanregung.

Hierzu kann grundlegend ein einheitlicher Farb-Wandler als Sensor verwendet werden. Denkbar ist aber auch, einen Aufbau wie in Fig. 1 oder Fig. 4 zu verwenden und eine zeitliche Separation zusätzlich zur Strahlaufteilung durch das dichroitische Prisma 300 vorzunehmen.

Bei Verwendung eines einheitlichen Farb-Wandlers kann das Licht, das auf den Farb-Wandler fällt, durch eine Bandsperre gefiltert werden, so dass keine Anregungsstrahlung auf den Farb-Wandler fällt (Anregungsstrahlungsfilter). Auf diese Weise wird möglich, über den Sensor sowohl ein Realbild in Form eines Farbbildes als auch ein Fluoreszenzbild zu detektieren.

Wie oben angegeben, können als optoelektronische Wandler 301, 302, 303 CCD-Chips oder CMOS-Bauelemente eingesetzt werden. Insbesondere bei Verwendung von CMOS-Sensoren kann eine effektive Fremdlichtunterdrückung erreicht werden, d.h. eine Unterdrückung solchen Lichts und solcher Strahlung, die nicht von der Beleuchtungseinheit 2 stammt, sondern von externen Lichtquellen der Umgebung, z. B. Tageslicht.

Grundlegend ist eine solche Fremdlichtunterdrückung auch mit CCD-Chips möglich.

Eine Fremdlichtunterdrückung wird durch ein so genanntes "Gating" erreicht, für dass es grundlegend zwei Anwendungsmöglichkeiten gibt:
1. Unterdrückung von Störlicht (Umgebungslicht);
2. Erzeugung des Realbildes und des Fluoreszenzbildes mit einer CCD-Anordnung für das Realbild und das Fluoreszenzbild im Zeitmultiplex.

Im Rahmen der ersten Möglichkeit kann ein Bildsensor in Form eines optoelektronischen Wandlers (analog zur Belichtungszeitauswahl bei einer Fotokamera) angesteuert werden, indem über einen Eingang am Sensor gesteuert wird, wann dieser das Signal aufnimmt. Bei Einsatz empfindlicher Sensoren (z. B. CMOS) kann dadurch die Belichtungszeit des Sensors reduziert werden, wodurch die Einflüsse von Störstrahlung unterdrückt werden und äußeres Umgebungslicht keinen Einfluss auf ein Fluoreszenzbild hat.

Bei der zweiten Variante kann die Aufnahme synchron mit den ebenfalls zu taktenden Lichtquellen für die Strahlung sichtbaren Lichts und die Fluoreszenzanregungsstrahlung gesteuert werden, wodurch abwechselnd ein Realbild und ein Fluoreszenzbild erhalten werden.

Um die Sensitivität der optoelektronischen Wandler zur Detektion der Infrarot- oder Nahinfrarotsignale zu erhöhen, kann auch ein so genanntes "Binning" eingesetzt werden, bei dem benachbarte Sensorzellen bei gleichzeitiger Reduzierung der Ortsauflösung zusammengeschlossen werden. Dadurch werden mehrere Bildpunkte der Sensoren miteinander verknüpft, indem die Signalwerte benachbarter Zellen addiert werden und dadurch ein höherer Signalwert für einen Ort erhalten wird. Die Fläche des detektierten Signalortes erhöht sich mit der Anzahl der Pixel, so dass die Ortsauflösung insgesamt abnimmt. Ein Vorteil dieser Methode gegenüber einer reinen Signalverstärkung liegt in der Mittelung des Grundrauschens.

Das Fluoreszenzsignal kann zusätzlich durch den Einsatz eines geeigneten Farbstoffs optimiert werden. Beispielsweise kann anstelle des Farbstoffs Indocyaningrün (ICG) eine Mischung aus ICG und Patent-Blau verwendet werden, um auf diese Weise mögliche Verunreinigungen durch den an sich unsichtbaren Fluoreszenzfarbstoff visuell sichtbar zu machen. Auf diese Weise können beispielsweise verunreinigte Tupfer sofort ausgetauscht werden, bevor sie bei einer Operation Verwendung finden.

Denkbar ist auch der Einsatz von kombinierten LED-Lichtquellen mit der Möglichkeit der Modulation, Taktung und Synchronisation mit der Aufnahmeeinheit 3.

Denkbar ist zudem, bei Verwendung eines dichroitischen Prismas 300 in seiner Drei-Kanal-Form oder Vier-Kanal-Form einen optoelektronischen Wandler durch einen Beleuchtungskanal zu ersetzen, so dass die beleuchtende Strahlung nicht über einen Diffusor oder endoskopischen Lichtleitanschluss dem Objekt 4 zugeführt wird, sondern direkt über das dichroitische Prisma 300. Am dichroitischen Prisma 300 kann hierfür ein Adapter vorhanden sein, der den Anschluss des Lichtleiters 24 der Beleuchtungseinheit 2 ermöglicht.

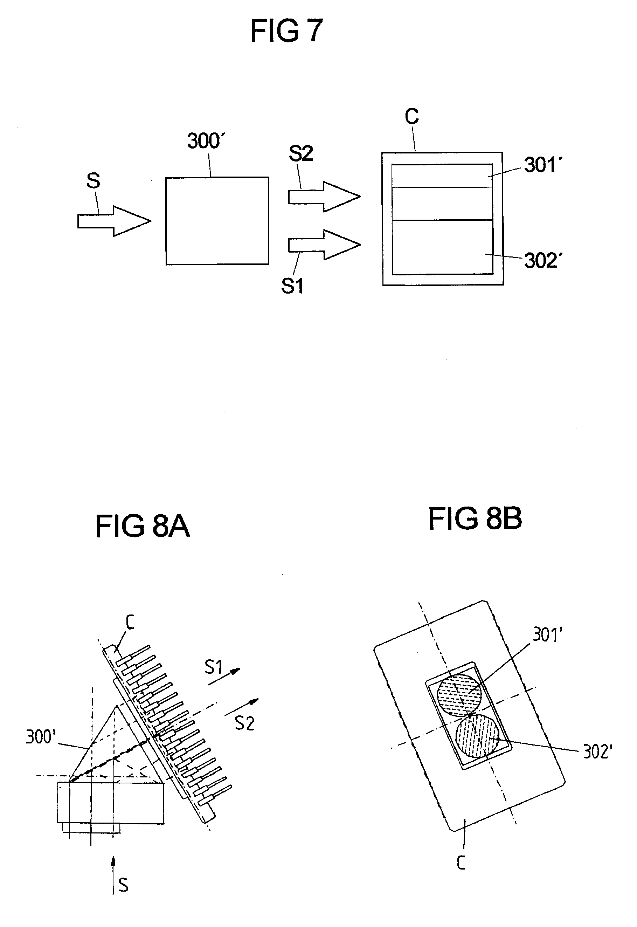

Fig. 7 und 8A, 8B zeigen schematisch eine Ausführungsform, bei dem ein optisches Signal S unter Verwendung eines dichroitischen Prismas 300' in Signalanteile S1, S2, entsprechend beispielsweise einem Signal sichtbaren Lichts und einem Fluoreszenzsignal, geteilt und durch einen zwei Teilbereiche 301', 302' aufweisenden einzigen optoelektronischen Wandler C in Form beispielsweise eines Farb-CCD-Chips (beispielsweise eines 16:9 HD-Chips) detektiert wird.

Wie in Fig. 7 schematisch und in Fig. 8A, 8B für eine konkrete Ausgestaltung dargestellt, wird das optische Signal S über ein dichroitisches Prisma 300' geteilt und auf verschiedene Bereiche des optoelektronischen Wandlers C abgebildet, wobei grundlegend zur Strahlteilung auch ein anderes dispersives Element, z. B. eine Spiegelanordnung oder eine andere Prismenanordnung, verwendbar ist.

Durch die Verwendung lediglich eines optoelektronischen Wandlers C kann zum einen die Detektorfläche des optoelektronischen Wandlers C effizient genutzt und zum anderen auch die Auswertung der detektierten Signale vereinfacht werden. Insbesondere ist die elektronische Verarbeitungskette für die nachgeschaltete Weiterverarbeitung der Bildinformationen wegen des einzig verwendeten optoelektronischen Wandlers C einsträngig.

Zusätzlich ergibt sich ein vorteilhaftes Bearbeitungsverfahren für die Zusammenführung der einzelnen Teilbilder. Denn die einander zugeordneten Pixel im Realbild und im Fluoreszenzbild liegen in derselben Zeile des optoelektronischen Wandlers und sind lediglich um eine bestimmte Pixelanzahl zueinander versetzt. In dem der Detektion eines Bildes folgenden Auslesevorgang unter Verwendung beispielsweise eines so genannten FPGAs oder DSPs können durch Speicherung der zuerst ausgegebenen Halbzeile und durch versetzte Ausgabe der Zeilenhälften pixelkonform die Bildinformationen parallel bearbeitet werden, so dass die Bildverarbeitung mit äußerst geringem Zeitverzug ablaufen kann. Insbesondere bei Echtzeitaufnahmen, die einem Arzt die Nachverfolgung eigener Instrumentenbewegungen ermöglichen sollen, ist dies von Vorteil.

Gegebenenfalls ist auch möglich, elektronisch eine Versatzkorrektur direkt nach dem optoelektronischen Wandler C oder in einer nachgeschalteten Steuer- und Verarbeitungseinheit vorzunehmen, mit der ein Versatz der Pixelorte ausgeglichen wird. Hintergrund ist hierbei, dass bei einem Auslesen von Halbzeilen toleranzbedingt die den Pixeln der Halbzeile zugeordneten Orte bezüglich des abgebildeten Objektes im Bild nicht genau übereinstimmen, so dass eine Versatzkorrektur erforderlich ist (ein bestimmter Pixel der dem Fluoreszenzsignal zugeordneten Halbzeile entspricht toleranzbedingt eventuell nicht demselben Objektdetail wie ein entsprechender Pixel der dem Signal sichtbaren Lichts zugeordneten Halbzeile). Um eine pixelkonforme, parallele Verarbeitung der Halbzeilen durchführen zu können, kann daher eine Versatzkorrektur erforderlich sein, die durch eine einfache Operation in der Art eines Schieberegisters in einer dem Bildsensor unmittelbar nachgeschalteten elektronischen Element vorteilhaft ausgeführt werden kann

Fig. 8A und 8B zeigen in einer Seitenansicht (Fig. 8A) und einer Draufsicht auf den optoelektronischen Wandler C (Fig. 8B) einen konkreten Aufbau, bei dem ein optoelektronischer Wandler C einem dichroitischen Prisma 300' nachgeschaltet ist und bei dem das dichroitische Prisma 300' das optische Signal S auf die Teilbereiche 301', 302' des optoelektronischen Wandlers C beispielsweise in Form eines CCD-Chips abbildet.

Bei der Anordnung gemäß Fig. 8A tritt die optische Strahlung S von unten in das dichroitische Prisma 300' ein und wird an einer Trennfläche des dichroitischen Prismas 300' teilweise reflektiert und teilweise transmittiert und damit in einen dem Signal sichtbaren Lichts S2 entsprechenden Anteil und einen dem Fluoreszenzsignal S1 entsprechenden Anteil getrennt. Dieses geschieht durch eine dichroitische Beschichtung der Trennfläche der Teilprismen des dichroitischen Prismas 300'.

Zusätzlich können Blockfilter eingesetzt werden, die auf der Eintrittsfläche des dichroitischen Prismas 300' aufgebracht oder in das gleichseitige dichroitische Prisma 300' eingebracht werden.

Bei der in Fig. 8A und 8B dargestellten Lösung ist eins der beiden Teilbilder gespiegelt, also seitenverkehrt. Durch die Ausführung eines Halbzeilenspeichers als LIFO-Speicher kann dies elegant korrigiert werden (bei einer alternativen Prismenanordnung ohne Spiegeleffekt müsste entsprechend ein FIFO-Speicher verwendet werden).

Wird als optoelektronischer Wandler ein Farb-CCD-Chip verwendet, muss die Berechnung der Farbanteile mit dem Überlagerungsvorgang der Halbzeilen in geeigneter Weise verbunden werden.

Selbstverständlich kann in diesem Zusammenhang auch ein optoelektronischer Wandler C mit drei Teilbereichen verwendet werden zur Umwandlung von drei unterschiedlichen Signalen (z.B. dem Fluoreszenzsignal S1, dem Signal sichtbaren Lichts S2 und dem Fluoreszenzanregungssignal S3).

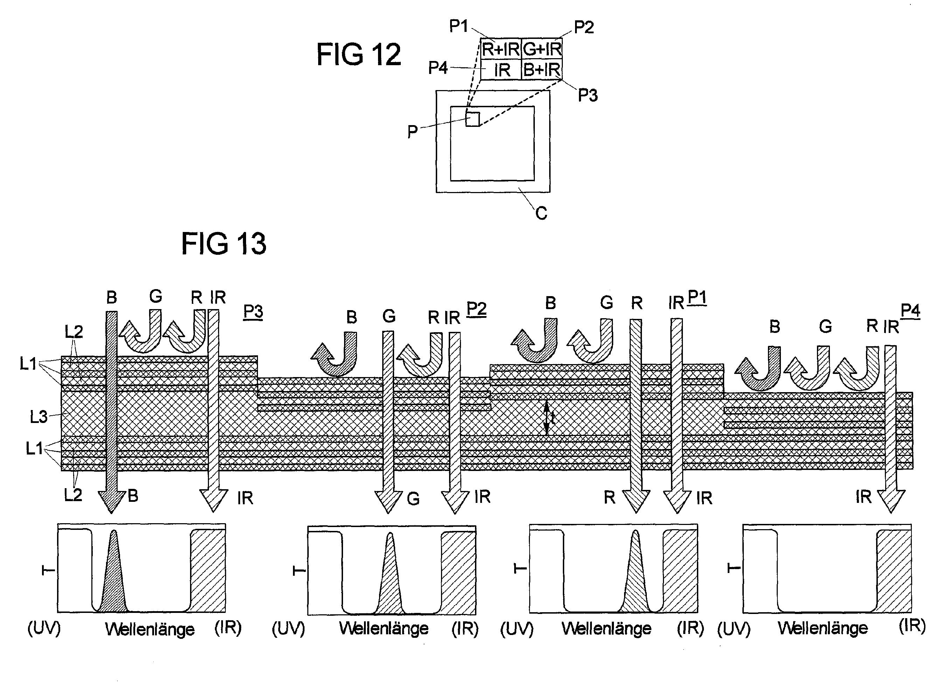

Fig. 12 zeigt in einer konkreten Ausgestaltung den Aufbau eines optoelektronischen Wandlers C, der eine Anzahl von regelmäßig in Zeilen und Spalten angeordneten Pixelelementen P aufweist, die jeweils aus einer Gruppe von vier Einzelpixeln P1-P4 bestehen. Wie in der schematischen Ansicht angedeutet dienen dabei drei Einzelpixel P1-P3 zur Umwandlung des Signals sichtbaren Lichts, indem ein erster Einzelpixel P1 für rotes Licht R, ein zweiter Einzelpixel P2 für grünes Licht G und ein dritter Einzelpixel P3 für blaues Licht B empfindlich ist. Die drei Einzelpixel P1-P3 sind zusätzlich auch empfindlich für Infrarotlicht IR, so dass die drei Einzelpixel P1-P3 zusammen mit einem vierten, ausschließlich für Infrarotlicht IR empfindlichen Einzelpixel P4 Infrarotlicht IR aufnehmen und umwandeln können.

Die Konfiguration der Einzelpixel P1-P4 zum Empfang von rotem, blauem oder grünen Licht R, G, B und/oder Infrarotlicht IR kann über eine mehrschichtige Filteranordnung erreicht werden, wie sie in Fig. 13 schematisch dargestellt ist. Dem eigentlichen Chip zum Empfangen der Lichtsignale sind dabei zur Ausbildung der Filteranordnung mehrere alternierende Schichten L1, L2 aus unterschiedlichen Materialien überlagert, wobei mittels einer die gesamte Bildsensorfläche überdeckenden Filterschicht L3 mit unterschiedlichen Schichtdicken t im Bereich der jeweiligen Einzelpixel P1-P4 ein spektraler Transmissionsbereich eingestellt wird.

Wie schematisch dargestellt, wird im Bereich des Einzelpixels P4 (links in Fig. 13) blaues Licht B in einem vorbestimmten Frequenzband durchgelassen und zusätzlich auch Infrarotlicht IR. Im Bereich des Einzelpixels P2 hingegen wird grünes Licht G und Infrarotlicht IR, und im Bereich des Einzelpixels P3 rotes Licht R und Infrarotlicht IR durchgelassen, um über den optoelektronischen Wandler C gewandelt zu werden. Im Bereich des Einzelpixels P4 schließlich kann ausschließlich Infrarotlicht IR die Filteranordnung passieren, so dass der Einzelpixel P4 nur für Infrarotlicht IR empfindlich ist.

Die zugehörigen Transmissionsspektren sind schematisch unterhalb der Schichtanordnung dargestellt und stellen qualitativ die Frequenzbereiche vom Ultraviolettlicht UV bis zum Infrarotlicht IR dar, in denen Licht die Schichtanordnung passieren kann.

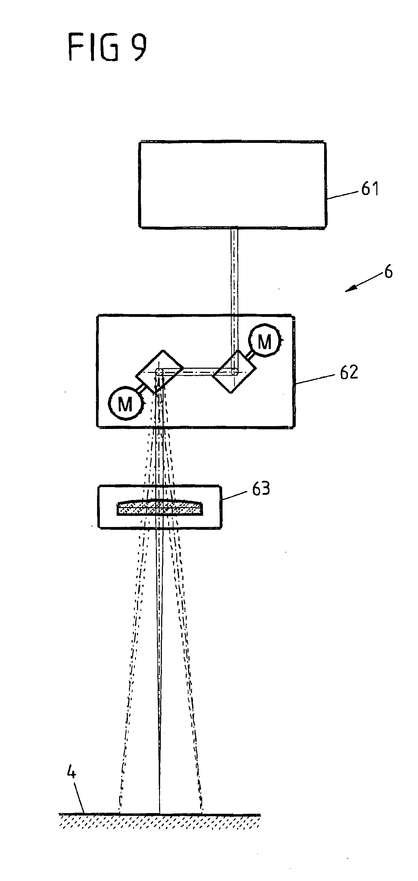

Fig. 9 bis 11 zeigen drei unterschiedliche Varianten von Vorrichtungen 6 zur Projektion eines aufgenommenen Fluoreszenzbildes auf die Oberflache eines Objektes 4. Die Grundidee hierbei ist, ein aufgenommenes Fluoreszenzbild unmittelbar auf dem Objekt 4, also beispielsweise auf der Haut- oder Gewebeoberfläche eines Patienten, und damit am Ort seiner Entstehung darzustellen.

Herkömmlich wird ein aufgenommenes Fluoreszenzbild, das als Videoausgabesignal V ausgegeben wird, auf einem Monitor dargestellt, auf dem das Fluoreszenzbild beispielsweise in Falschfarbe einem Realbild überlagert wird. Dies hat zur Folge, dass ein begutachtender Arzt das Fluoreszenzbild ausschließlich auf dem Monitor betrachten kann, was bei einer endoskopischen Operationstechnik keine Einschränkung ist, bei einer offenchirurgischen Ausführung einer Operation an einem Patienten jedoch umständlich für den Arzt sein kann.

In Abkehr hiervon kann mittels der Vorrichtung 6 ein aufgenommenes Fluoreszenzbild in Echtzeit unmittelbar an dem Ort dargestellt werden, an dem es aufgenommen worden ist. Weitere optische Hilfsmittel, wie z. B. ein Monitor, sind nicht erforderlich.

Mittels der Vorrichtung 6 wird ein aufgenommenes Fluoreszenzbild mit Hilfe eines Projektors oder eines Zweiachsenscanners (so genannter X/Y-Scanner) auf das Objekt 4 projiziert. Die in Fig. 9 dargestellte Vorrichtung verwendet beispielsweise einen X/Y-Scanner 62, der mit Hilfe von mechanischen Motoren und Spiegelanordnungen einen von einer Lichtquelle 61 (beispielsweise ein Laser) erzeugten Lichtstrahl über ein Objektiv 63 auf das Objekt 4 projiziert, indem mittels des X/Y-Scanners 62 ein intensitätsmodulierter Strahl der Lichtquelle 61 (mit sichtbarem Licht) periodisch über den Untersuchungsbereich des Objektes 4 bewegt wird, so dass dort ein dem Fluoreszenzbild entsprechendes Indikatorbild entsteht.

Die Modulationsfunktion der Laserstrahlleistung kann mit dem lokalen Fluoreszenzsignal beispielsweise monoton ansteigend sein. Alternativ kann die Charakteristik der Modulationsfunktion auch zweiwertig (0/1 unter Verwendung eines Schwellwertes), linear proportional oder logarithmisch proportional ausgeführt sein.

Zusätzlich kann die exakte Positionierung des Fluoreszenzbildes auf dem Objekt 4, die aufgrund der zumeist unebenen Oberfläche des Objekts 4 gestört sein kann, z.B. durch Rückkopplung verbessert werden und/oder Unterschiede des lokalen Reflektionsvermögens, die das Indikatorbild gegebenenfalls verfälschen, können erfasst und in der Modulationsfunktion korrigiert werden.

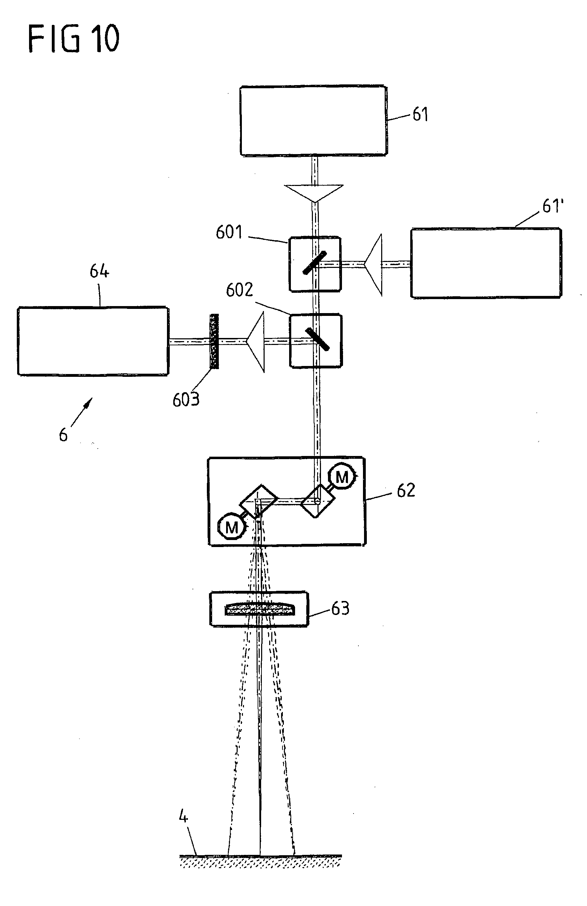

Eine Ausführungsform einer Vorrichtung 6, bei der nicht nur ein Fluoreszenzbild auf dem Objekt 4 abgebildet, sondern gleichzeitig auch ein Signal aus dem Bereich des Objektes 4 aufgenommen wird, ist in Fig. 10 dargestellt. Bauteile gleicher Funktion sind dabei mit gleichen Bezugszeichen wie bisher versehen.

Die Vorrichtung gemäß Fig. 10 dient einerseits zur Projektion und Darstellung des aufgenommenen Fluoreszenzbildes, andererseits aber auch zur Aufnahme und kann hierzu dem Kamerakopf 30 der Aufnahmeeinheit 3 des Bildgebungssystems 1 gemäß Fig. 1 oder Fig. 4 vorgeschaltet sein.

Bei der Ausführungsform gemäß Fig. 10 erzeugt eine Lichtquelle 61' eine Fluoreszenzanregungsstrahlung in einem Wellenlängenbereich zwischen beispielsweise 700 nm und 800 nm, die über einen Strahlkombinierer 601, einen Strahlteiler 602, einen X/Y-Scanner 62 und ein Objektiv 63 zur Anregung eines Fluoreszenzsignals auf das Objekt 4 gestrahlt wird. Hierzu wird mittels des X/Y-Scanners 62 der Strahl über das Objekt 4 geführt und das Objekt 4 damit pixelweise angeregt.

Synchron wird das rückgestrahlte Fluoreszenzsignal über das Objektiv 63 und den X/Y-Scanner 62 sowie den Strahlteiler 602 und einen Filter 603 hin zu einem Detektor 64 geleitet. Die Aufnahme erfolgt ebenfalls pixelweise, wobei das Fluoreszenzsignal aus dem von der Lichtquelle 61' gerade angeregten Pixel erfasst wird.

Das so empfangene Fluoreszenzsignal wird umgewandelt und in Echtzeit über die Lichtquelle 61 als Falschfarbensignal zur Generierung des Indikatorbildes beziehungsweise des betreffenden Pixels des Indikatorbildes auf dem Objekt 4 ausgestrahlt. Der von der Lichtquelle 61 erzeugte Strahl wird dabei mittels des Strahlkombinierers 601 mit dem Strahl der Lichtquelle 61' kombiniert und ebenfalls über den X/Y-Scanner 62 und das Objektiv 63 auf das Objekt 4 gelenkt. Auf diese Weise wird auf dem Objekt 4 unmittelbar am Ort der Aufnahme in Echtzeit eine sichtbare Indikation des lokalen Fluoreszenzsignals dargestellt. Durch die schnelle Scannbewegung sieht der Betrachter auf der Gewebeoberfläche unmittelbar ein dem Fluoreszenzbild in Falschfarbe entsprechendes Indikatorbild.

Folgende Vorteile bestehen bei der Ausführungsform gemäß Fig. 10:
- es gibt keinen Bildauswertestrang, sondern nur einen einzelnen Signalverarbeitungskanal;
- eine Zwischenspeicherung von Bilddaten ist nicht notwendig;
- eine Separierung eines Fluoreszenzanregungssignals gegenüber einem Signal sichtbaren Lichts ist nicht notwendig, da ein Realbild nur im Auge des Betrachtes entsteht, das für die Fluoreszenzanregungsstrahlung nicht empfindlich ist;
- der Detektor 64 kann in einer bevorzugten Ausführungsform großflächig aufgebaut sein und muss keine Bewegung ausführen, da die Ortsinformation durch die aktuelle Position des X/Y-Scanners 62 vorgegeben ist;
- es ergibt sich ein optisch einfacher Aufbau mit hoher Empfindlichkeit im Fluoreszenzkanal, da die Detektorfläche des Detektors 64 groß gewählt werden kann;
- als Detektoren können einfache PIN-Dioden mit vorgesetztem Filter gegen unerwünschte Strahlanteile verwendet werden;
- X/Y-Scanner sind kostengünstig verfügbar;
- als Markierungslaser kann z. B. ein grüner Laser verwendet werden, die ebenfalls kostengünstig verfügbar sind.

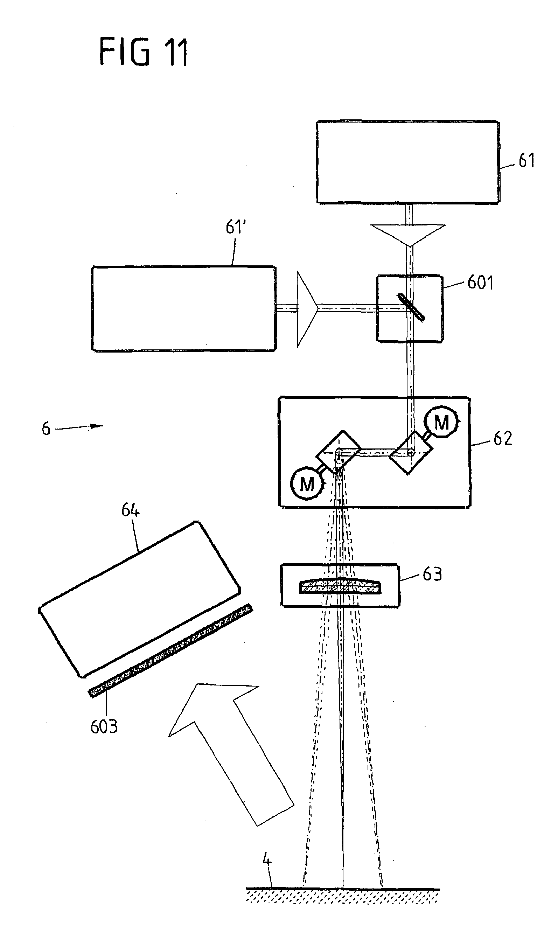

In einer in Fig. 11 dargestellten, anderen Ausgestaltung kann ein resultierendes Fluoreszenzsignal auch über einen Detektor 64 in Form eines flächigen CCD-Chips empfangen werden. Wie vorangehend anhand von Fig. 10 beschrieben, wird dabei mittels einer Lichtquelle 61' das Objekt 4 angeregt, indem ein emittierter Strahl mittels des X/Y-Scanners 62 über das Objekt 4 geführt wird. Dadurch wird das Objekt 4 sukzessive angeregt, so dass sich eine flächige Anregung ergibt, aus deren Bereich der Detektor 64 das Fluoreszenzsignal aufnimmt. Das so aufgenommene Fluoreszenzbild kann wiederum über die Lichtquelle 61 und über den Strahlkombinierer 601 sowie den X/Y-Scanner 62 auf das Objekt 4 projiziert werden.

Die Vorrichtung 6 gemäß den Ausführungsformen in Fig. 9 bis 11 kann vorteilhafterweise als kompaktes, handgehaltenes Gerät ausgeführt werden und ist mit modernen Akkumulatoren auch kabellos betreibbar.

Die in Fig. 9 bis 11 dargestellten Ausführungsformen einer Vorrichtung zur Projektion sind grundsätzlich auch unabhängig von den in Fig. 1 bis 5 beschriebenen Bildgebungssystemen verwendbar und können daher auch als eigenständige Erfindungen betrachtet werden.

### Bezugszeichenliste

- 1: Bildgebungssystem
- 2: Beleuchtungseinheit
- 21, 22: Lichtquelle
- 23: Koppelelement
- 24: Lichtleiter
- 240, 241: Anschluss
- 3: Aufnahmeeinheit
- 30: Kamerakopf
- 300, 300': Dichroitisches Prisma
- 301-303: Optoelektronischer Wandler
- 301', 302': Teilbereich
- 304: Absorberelement
- 305: Anschluss
- 306, 307, 310: Analog/Digital-Wandler
- 308, 309, 311: Treiber
- 31: Objektiv
- 32: Endoskop
- 320, 321: Kanal
- 4: Objekt
- 5: Steuer- und Verarbeitungseinheit
- 501, 502, 504: Datenkabel
- 503: Anschluss
- 511, 512, 516: Vorverarbeitungseinheit
- 513: Bildverarbeitungseinheit
- 514: Endverarbeitungseinheit
- 515: Anschluss
- 52: Steuereinheit
- 6: Projektoreinheit
- 601: Strahlkombinierer
- 602: Strahlteiler
- 603: Filter
- 61, 61': Strahlungsquelle
- 62: Zweiachsenscanner
- 63: Objektiv
- 64: Detektor
- A1-A3: Teilprisma
- A1'-A5': Teilprisma
- B: Blaues Licht
- C: CCD-Chip
- D1, D2, D3: Elektronisches Datensignal
- F1, F2: Dichroitisches Filter
- F': Filter
- F1'-F3': Dichroitisches Filter
- G: Grünes Licht
- IR: Infrarotlicht
- L1, L2, L3: Schicht
- P: Pixelelement
- P1-P4: Einzelpixel
- R: Rotes Licht
- S: Optisches Signal
- S1: Fluoreszenzsignal
- S2: Signal sichtbaren Lichts
- S3: Fluoreszenzanregungssignal
- S1'-S4': Signal
- t: Schichtdicke
- UV: Ultraviolettlicht
- V: Videoausgabesignal

## Patentansprüche

1. Bildgebungssystem zur fluoreszenz-optischen Visualisierung eines zweidimensionalen oder dreidimensionalen Objekts (4) in Form von Organen, Organellen oder Gewebebereichen eines Patienten für medizinische Zwecke, mit
- einer Beleuchtungseinheit (2), die ausgebildet und vorgesehen ist, optische Strahlung in einem vorbestimmten Wellenlängenbereich zur Beleuchtung des Objekts (4) und zur Anregung eines in dem Objekt (4) enthaltenen fluoreszierenden Stoffes zu emittieren,
- einer Aufnahmeeinheit (3), die ausgebildet und vorgesehen ist, ein optisches Signal (S) aus dem Bereich des Objekts (4) aufzunehmen und das optische Signal (S) in ein Fluoreszenzsignal (S1) mit einem ersten Wellenlängenbereich und ein Signal sichtbaren Lichts (S2) mit einem zweiten Wellenlängenbereich aufzuteilen,
wobei die optische Aufnahmeeinheit (3) einen mehrere Teilbereiche (301', 302') aufweisenden optoelektronischen Wandler (C) zur Umwandlung des Fluoreszenzsignals (S1) in ein erstes elektronisches Datensignal (D1) und des Signals sichtbaren Lichts (S2) in ein zweites elektronisches Datensignal (D2) aufweist,
wobei die optische Aufnahmeeinheit (3) ein dichroitisches Prisma (30) zur Aufteilung des aufgenommenen optischen Signals (S) in das Fluoreszenzsignal (S1) und das Signal sichtbaren Lichts (S2) aufweist,
**dadurch gekennzeichnet,**
**dass** der mit dem dichroitischen Prisma (30) verbundene optoelektronische Wandler (C) unmittelbar auf dem dichroitischen Prisma (30) angeordnet und so mit dem dichroitischen Prisma (30) verbunden ist.

2. Bildgebungssystem nach Anspruch 1, **dadurch gekennzeichnet, dass** von den Teilbereichen (301', 302') des optoelektronischen Wandlers (C) ein erster Teilbereich (301') das Fluoreszenzsignal (S1) in ein erstes elektronisches Datensignal (D1) und ein zweiter Teilbereich (302') das Signal sichtbaren Lichts (S2) in ein zweites elektronisches Datensignal (D2) umwandelt.

3. Bildgebungssystem nach Anspruch 1 oder 2, **dadurch gekennzeichnet, dass** der erste Wellenlängenbereich des Fluoreszenzsignals (S1) Wellenlängen größer als 800 nm und der zweite Wellenlängenbereich des Signals sichtbaren Lichts (S2) Wellenlängen kleiner als 700 nm enthält.

4. Bildgebungssystem nach einem der vorangehenden Ansprüche, **dadurch gekennzeichnet, dass** der optoelektronische Wandler (C) eine Anzahl von Pixelelementen (P) aufweist, die jeweils aus vier Einzelpixeln (P1-P4) bestehen, wobei drei Einzelpixel (P1-P3) zur Umwandlung des Signals sichtbaren Lichts (S2) und ein Einzelpixel (P4) zur Umwandlung des Fluoreszenzsignals (S1) ausgebildet sind.

5. Bildgebungssystem nach Anspruch 4, **dadurch gekennzeichnet, dass** die drei Einzelpixel (P1-P3) zur Umwandlung des Signals sichtbaren Lichts (S2) zusätzlich auch zur Umwandlung des Fluoreszenzsignals (S1) ausgebildet sind.

6. Bildgebungssystem nach einem der vorangehenden Ansprüche, **dadurch gekennzeichnet, dass** das aufgenommene optische Signal (S) zusätzlich in ein Fluoreszenzanregungssignal (S3) mit einem dritten Wellenlängenbereich geteilt wird, der von dem ersten Wellenlängenbereich und dem zweiten Wellenlängenbereich unterschiedlich ist.

7. Bildgebungssystem nach Anspruch 6, **dadurch gekennzeichnet, dass** das Fluoreszenzanregungssignal (S3) einen Wellenlängenbereich zwischen 700 nm und 800 nm aufweist.

8. Bildgebungssystem nach Anspruch 6 oder 7, **dadurch gekennzeichnet, dass** der mehrere Teilbereiche (301', 302') aufweisende optoelektronische Wandler (C) einen dritten Teilbereich (301', 302') aufweist, der das Fluoreszenzanregungssignal (S3) in ein drittes elektronisches Datensignal (D3) umwandelt.

9. Bildgebungssystem nach Anspruch 6 oder 7, **dadurch gekennzeichnet, dass** ein Teilbereich (302') des mehrere Teilbereiche (301', 302') aufweisenden optoelektronischen Wandlers (C) sowohl das Signal sichtbaren Lichts (S2) als auch das Fluoreszenzanregungssignal (S3) umwandelt.

10. Bildgebungssystem nach einem der vorangehenden Ansprüche, **dadurch gekennzeichnet, dass** die Aufnahmeeinheit (3) mit einem Objektiv (31) zur Aufnahme des optischen Signals (S) außerhalb des Objekts (4) und/oder mit einer Aufnahmeeinrichtung (32) zur Aufnahme des optischen Signals (S) aus dem Inneren des Objekts (4) verbindbar ist.

11. Bildgebungssystem nach einem der vorangehenden Ansprüche, **dadurch gekennzeichnet, dass** die Beleuchtungseinheit (2) zwei Lichtquellen (21, 22) aufweist, von denen eine erste Lichtquelle (21) Fluoreszenzanregungsstrahlung und eine zweite Lichtquelle (22) Strahlung im Bereich sichtbaren Lichts erzeugt.

12. Bildgebungssystem nach einem der vorangehenden Ansprüche, **dadurch gekennzeichnet, dass** die Beleuchtungseinheit (2) ein optisches Koppelelement (23) zur Kopplung der Fluoreszenzanregungsstrahlung und der Strahlung im Bereich sichtbaren Lichts aufweist und die so erzeugte, gekoppelte optische Strahlung in einen Lichtleiter (24) zur Beleuchtung des Objekts (4) einkoppelt.

13. Bildgebungssystem nach Anspruch 12, **dadurch gekennzeichnet, dass** der Lichtleiter (24) mit einem Element zur Anpassung der Abstrahlungscharakteristik der optischen Strahlung zur Beleuchtung des Objektes (4) von außen oder zur Beleuchtung von Bereichen innerhalb des Objektes (4) verbindbar ist.

## Claims

1. An imaging system for the fluorescence-optical-visualization of a two-dimensional or three-dimensional object (4) in form of organs, organelles or tissue regions of a patient for medical purposes, comprising
- an illumination unit (2), which is designed and provided for emitting optical radiation in a predetermined wavelength range in order to illuminate the object (4) and excite a fluorescent substance contained in the object (4),
- a capturing unit (3), which is designed and provided for capturing an optical signal (S) from the region of the object (4) and for splitting the optical signal (S) into a fluorescence signal (S1) having a first wavelength range and a signal of visible light (S2) having a second wavelength range,
wherein the optical capturing unit (3) has an optoelectronic converter (C) having a plurality of partial regions (301', 302') and serving for converting the fluorescence signal (S1) into a first electronic data signal (D1) and the signal of visible light (S2) into a second electronic data signal (D2),
wherein the optical capturing unit (3) has a dichroic prism (30) for splitting the captured optical signal (S) into the fluorescence signal (S1) and the signal of visible light (S2),
**characterized**
**in that** the optoelectronic converter (C) connected to the dichroic prism (30) is arranged directly on the dichroic prism (30) and thus connected to the dichroic prism(30).

2. The imaging system as claimed in claim 1, **characterized in that** of the partial regions (301', 302') of the optoelectronic converter (C) a first partial region (301') converts the fluorescence signal (S1) into a first electronic data signal (D1) and a second partial region (302') converts the signal of visible light (S2) into a second electronic data signal (D2).

3. The imaging system as claimed in claim 1 or 2, **characterized in that** the first wavelength range of the fluorescence signal (S1) contains wavelengths of greater than 800 nm and the second wavelength range of the signal of visible light (S2) contains wavelengths of less than 700 nm.

4. The imaging system as claimed in any of the preceding claims, **characterized in that** the optoelectronic converter (C) has a number of pixel elements (P) each consisting of four individual pixels (P1-P4), three individual pixels (P1-P3) being designed for converting the signal of visible light (S2) and one individual pixel (P4) being designed for converting the fluorescence signal (S1).

5. The imaging system as claimed in claim 4, **characterized in that** the three individual pixels (P1-P3) for converting the signal of visible light (S2) are additionally also designed for converting the fluorescence signal (S1).

6. The imaging system as claimed in any of the preceding claims, **characterized in that** the captured optical signal (S) is additionally split into a fluorescence excitation signal (S3) having a third wavelength range, which differs from the first wavelength range and the second wavelength range.

7. The imaging system as claimed in claim 6, **characterized in that** the fluorescence excitation signal (S3) has a wavelength range of between 700 nm and 800 nm.

8. The imaging system as claimed in claim 6 or 7, **characterized in that** the optoelectronic converter (C) having a plurality of partial regions (301', 302') has a third partial region (301', 302'), which converts the fluorescence excitation signal (S3) into a third electronic data signal (D3).

9. The imaging system as claimed in claim 6 or 7, **characterized in that** a partial region (302') of the optoelectronic converter (C) having a plurality of partial regions (301', 302') converts both the signal of visible light (S2) and the fluorescence excitation signal (S3).

10. The imaging system as claimed in any of the preceding claims, **characterized in that** the capturing unit (3) can be connected to a lens (31) for capturing the optical signal (S) outside the object (4) and/or to a capturing device (32) for capturing the optical signal (S) from the interior of the object (4).

11. The imaging system as claimed in any of the preceding claims, **characterized in that** the illumination unit (2) has two light sources (21, 22) of which a first light source (21) generates fluorescence excitation radiation and a second light source (22) generates radiation in the range of visible light.

12. The imaging system as claimed in any of the preceding claims, **characterized in that** the illumination unit (2) has an optical coupling element (23) for coupling the fluorescence excitation radiation and the radiation in the range of visible light and couples the coupled optical radiation thus generated into an optical waveguide (24) for illuminating the object (4).

13. The imaging system as claimed in claim 12, **characterized in that** the optical waveguide (24) can be connected to an element for adapting the emission characteristic of the optical radiation for illuminating the object (4) from outside or for illuminating regions within the object (4).

## Revendications

1. Système d'imagerie pour la visualisation par optique de fluorescence d'un objet (4) bidimensionnel ou tridimensionnel sous forme d'organes, d'organites ou de zones de tissu d'un patient à des fins médicales, comprenant :
- une unité d'éclairage (2) qui est conçue et prévue pour émettre un rayonnement optique dans un domaine de longueurs d'onde prédéfini pour l'éclairage de l'objet (4) et pour l'excitation d'un matériau fluorescent contenu dans l'objet (4),
- une unité de réception (3) qui est conçue et prévue pour recevoir un signal optique (S) à partir la zone de l'objet (4) et pour diviser le signal optique (S) en un signal de fluorescence (S1) ayant un premier domaine de longueurs d'onde et en un signal de lumière visible (S2) ayant un deuxième domaine de longueurs d'onde,
l'unité de réception optique (3) comprenant un convertisseur optoélectronique (C) comprenant plusieurs zones partielles (301', 302') pour la conversion du signal de fluorescence (S1) en un premier signal de données électronique (D1) et du signal de lumière visible (S2) en un deuxième signal de données électronique (D2),
l'unité de réception optique (3) comprenant un prisme dichroïque (30) pour la division du signal optique (S) reçu en signal de fluorescence (S1) et en signal de lumière visible (S2),
**caractérisé**
**en ce que** le convertisseur optoélectronique (C) relié au prisme dichroïque (30) est disposé directement sur le prisme dichroïque (30) et est relié de cette façon au prisme dichroïque (30).

2. Système d'imagerie selon la revendication 1, **caractérisé en ce que**, parmi les zones partielles (301', 302') du convertisseur optoélectronique (C), une première zone partielle (301') convertit le signal de fluorescence (S1) en un premier signal de données électronique (D1) et une deuxième zone partielle (302') convertit le signal de lumière visible (S2) en un deuxième signal de données électronique (D2).

3. Système d'imagerie selon la revendication 1 ou 2, **caractérisé en ce que** le premier domaine de longueurs d'onde du signal de fluorescence (S1) contient des longueurs d'onde supérieures à 800 nm et le deuxième domaine de longueurs d'onde du signal de lumière visible (S2) des longueurs d'onde inférieures à 700 nm.

4. Système d'imagerie selon l'une quelconque des revendications précédentes, **caractérisé en ce que** le convertisseur optoélectronique (C) comprend un certain nombre de blocs de pixels (P) qui sont composés chacun de quatre pixels (P1-P4), trois pixels (P1-P3) étant conçus pour la conversion du signal de lumière visible (S2) et un pixel (P4) étant conçu pour la conversion du signal de fluorescence (S1).

5. Système d'imagerie selon la revendication 4, **caractérisé en ce que** les trois pixels (P1-P3) pour la conversion du signal de lumière visible (S2) sont conçus en plus également pour la conversion du signal de fluorescence (S1).

6. Système d'imagerie selon l'une quelconque des revendications précédentes, **caractérisé en ce que** le signal optique (S) reçu est divisé en plus en un signal d'excitation de fluorescence (S3) ayant un troisième domaine de longueurs d'onde qui est différent du premier domaine de longueurs d'onde et du deuxième domaine de longueurs d'onde.

7. Système d'imagerie selon la revendication 6, **caractérisé en ce que** le signal d'excitation de fluorescence (S3) comprend un domaine de longueurs d'onde entre 700 nm et 800 nm.

8. Système d'imagerie selon la revendication 6 ou 7, **caractérisé en ce que** le convertisseur optoélectronique (C) comprenant plusieurs zones partielles (301', 302') comprend une troisième zone partielle (301', 302') qui convertit le signal d'excitation de fluorescence (S3) en un troisième signal de données électronique (D3).

9. Système d'imagerie selon la revendication 6 ou 7, **caractérisé en ce qu'**une zone partielle (302') du convertisseur optoélectronique (C) comprenant plusieurs zones partielles (301', 302') convertit aussi bien le signal de lumière visible (S2) que le signal d'excitation de fluorescence (S3).

10. Système d'imagerie selon l'une quelconque des revendications précédentes, **caractérisé en ce que** l'unité de réception (3) peut être reliée à un objectif (31) pour la réception du signal optique (S) en dehors de l'objet (4) et/ou à un système de réception (32) pour la réception du signal optique (S) depuis l'intérieur de l'objet (4).

11. Système d'imagerie selon l'une quelconque des revendications précédentes, **caractérisé en ce que** l'unité d'éclairage (2) comprend deux sources de lumière (21, 22) parmi lesquelles une première source de lumière (21) génère un rayonnement d'excitation de fluorescence et une seconde source de lumière (22) génère un rayonnement dans le domaine de la lumière visible.

12. Système d'imagerie selon l'une quelconque des revendications précédentes, **caractérisé en ce que** l'unité d'éclairage (2) comprend un élément de couplage optique (23) pour le couplage du rayonnement d'excitation de fluorescence et du rayonnement dans le domaine de la lumière visible et **en ce que** le rayonnement optique, généré et couplé de cette façon, est couplé dans un guide d'ondes optiques (24) pour l'éclairage de l'objet (4).

13. Système d'imagerie selon la revendication 12, **caractérisé en ce que** le guide d'ondes optiques (24) peut être relié à un élément pour l'adaptation de la caractéristique de rayonnement du rayonnement optique pour l'éclairage de l'objet (4) de l'extérieur ou pour l'éclairage de zones à l'intérieur de l'objet (4).
